# EUROPEAN PATENT APPLICATION

(11) **EP 4 480 489 A1**
(43) Date of publication of application: **25.12.2024**
(21) Application number: 23305996.3
(22) Date of filing: 22.06.2023
(51) Int. Cl.: A61K 38/00, A61K 38/18, C12P 21/00

(54) **COMPOSITION**

(71) Applicant: Kyron.bio SAS, 75008 Paris (FR)
(72) Inventor: MCLAUGHLIN, Emilia Jane, 75008 PARIS (FR); LOCHHEAD, Marina Rose, 75008 PARIS (FR)
(74) Representative: Gill Jennings & Every LLP

(57) **Abstract**

The present invention involves utilising oligosaccharyltransferase (OST) enzymes obtained from *Trypanosoma brucei (T. brucei)* to improve the expression of functional and stable recombinant proteins in a mammalian expression system, which includes creating novel engineered mammalian cell lines and genetic constructs comprising the nucleotides encoding the OST enzymes obtained from *T. brucei.*

## Description

### FIELD OF THE INVENTION

The present invention involves utilising oligosaccharyltransferase (OST) enzymes obtained from *Trypanosoma brucei (T. brucei)* to improve the expression of functional and stable recombinant proteins in a mammalian expression system, which includes creating novel engineered mammalian cell lines and genetic constructs comprising the nucleotides encoding the OST enzymes obtained from *T. brucei.*

### BACKGROUND

The addition of glycan moieties to the surface of proteins, called glycosylation, is one of the most common posttranslational modifications (PTMs) and is a natural process that is important for the function and stability of many proteins. This complex process begins in the endoplasmic reticulum where a glycan moiety is added to a newly synthesized protein. This will in turn influence the structure of the protein and the protein's biological activity. Indeed, the inherent structural variations of glycan moieties can modulate the properties of a protein. As such, the production of therapeutic glycoproteins in industry is of great interest. Producing proteins with added glycan moieties has potential to improve yield and stability of therapeutic proteins and also allow for the modulation of activity and new drug discovery.

Protein glycosylation is a naturally occurring process and in the human body approximately 50% of proteins are glycosylated, and therefore are referred to as glycoproteins. In the pharmaceutical industry, two-thirds of the regulatory approved therapeutic proteins are glycoproteins (Delobel, Mass Spectrometry of Glycoproteins, pp 1-21, 2021), which all have at least one glycan moiety on their surface (which is a particular sugar molecule), but some have dozens of glycan moieties attached. These glycan moieties are fundamentally important for the functioning of the protein - they affect the activity of the protein, how long it is stable, and how well-folded the protein is.

There are two types of glycosylation: N-glycosylation and O-glycosylation. Over 90% of glycoproteins are modified by N-glycosylation (Helenius, Mol Biol Cell, 5(3);253-65, 1994). In mammals, N-glycosylation is the covalent attachment of Glc₃MAN₉GlcNAc₂ to asparagine (Asn or N) residues on the polypeptide chain, which lie within an Asn-X-Ser/Thr motif, wherein X can be any amino acid except proline (Reilly et al., Nature Reviews Nephrology, 15(346-366), 2019). This can occur both co-translationally or post-translationally (Canada et al., Cell, 136(2): 272-283, 2010) within the endoplasmic reticulum (ER) of the cell (Kleizen & Braakman, Curr Opin Cell Biol, 16(4):343-9, 2004).

The addition of an N-glycan to a protein is catalysed by a multi-subunit enzyme known as an oligosaccharyltransferase (OST or also OTase). The mammalian OST is a multi-subunit membrane protein, including two different catalytic subunits (STT3A and STT3B), along with at least six other non-catalytic subunits whose function is poorly studied (Mohanty et al., Biomolecules, 10(4):624, 2020), (Pfeffer et al., Nature Communications, 5 (3072), 2014). The two catalytic subunits have distinct substrate specificities (Cherepanova & Gilmore, Scientific Reports, 6(20946), 2016), and the entire OST catalyses the *en bloc* transfer of a pre-assembled high mannose oligosaccharide onto asparagine residues (Kheller & Gilmore, Glycobiology, 16(4):47-62, 2005).

However, in eukaryotes in general, the OSTs that carry out glycosylation are inefficient, with around 35% of N-glycan sequons unoccupied (Petrescu et al., Glycobiology, 14(2):103-14, 2004). This represents a major limitation in industry where mammalian cells are commonly used for bioproduction of glycosylated proteins, including therapeutic proteins. Indeed, N-glycan site occupancy is one of the key quality attributes that is analysed by the Food and Drug Administration (FDA) for the regulatory approval of therapeutic proteins. This is because N-glycosylation plays an important role in numerous aspects of protein production, including protein yield, protein function, correct protein folding, and controlling batch to batch variation.

Accordingly, there is a need in the art to improve the production of proteins that are recombinantly produced in mammalian cell lines. Specifically, there is a need to enhance the N-glycan site occupancy of recombinant proteins in order to substantially improve the yield, activity, stability, and reproducibility of recombinant proteins in the drug production and discovery industry.

### SUMMARY OF INVENTION

The inventors of the present invention have surprisingly found that specific OST enzymes that originate from the *Trypanosoma brucei* parasite can be engineered into a mammalian bioproduction cell in order to significantly improve the N-glycan profile of recombinant proteins produced in mammalian cells. This holds the potential to improve the yield, stability and activity of recombinant proteins produced.

In a first aspect of the invention, there is provided a mammalian cell comprising at least one nucleic acid sequence encoding at least one oligosaccharyltransferase (OST) protein or functional fragment thereof, wherein the at least one OST protein is a *Trypanosoma spp.* OST protein, preferably wherein the at least one OST protein is a *Trypanosoma brucei* OST protein.

In a second aspect of the invention, there is provided a mammalian cell comprising at least one nucleic acid sequence encoding at least one oligosaccharyltransferase (OST) protein or functional fragment thereof, wherein the at least one nucleic acid sequence encoding the at least one OST protein or functional fragment thereof comprises a sequence according to any of SEQ ID NOs: 1, 2, 3, 4, 5, 6, 7, 8, 9, 10, 11, 12, 13, 21 and/or 22, or a sequence having at least 70% sequence identity thereto. In a preferred embodiment, the mammalian cell may comprise a combination of the nucleic acids, such as the combinations set out in the detailed description.

In a third aspect of the invention, there is provided a mammalian cell comprising at least one nucleic acid sequence encoding at least one oligosaccharyltransferase (OST) protein or functional fragment thereof, wherein the at least one OST protein comprises an amino acid sequence according to any of SEQ ID NOs: 14, 15, 16, 17, 18, 19, and/or 20 or a sequence having at least 70% sequence identity thereto. In a preferred embodiment, the mammalian cell may comprise a combination of the nucleic acids, such as the combinations set out in the detailed description.

In a fourth aspect of the invention, there is provided an isolated nucleic acid molecule comprising a sequence according to any of SEQ ID NOs: 1, 2, 3, 4, 5, 6, 7, 8, 9, 10, 11, 12, 13, 21, and/or 22, or a sequence having at least 70% identity thereto, preferably wherein the isolated nucleic acid molecule comprises a sequence according to SEQ ID NOs: 3, 5, 7, 9, 11, and/or 13.

In a fifth aspect of the invention, there is provided a nucleic acid vector comprising (i) at least one nucleic acid sequence according to any of SEQ ID NOs: 1, 2, 3, 4, 5, 6, 7, 8, 9, 10, 11, 12, 13, 21 and/or 22, or a sequence having at least 70% identity thereto; or (ii) at least one isolated nucleic acid molecule according to the fourth aspect of the invention, preferably wherein the nucleic acid vector is selected from the group comprising, but not limited to, a plasmid-based expression vector, a bacterial artificial chromosome (BAC) vector, and a viral vector such as an adenoviral vector, adeno-associated vector (AAV), retroviral vector or lentiviral vector. In a preferred embodiment, the vector may comprise a combination of the nucleic acids, such as the combinations set out in the detailed description.

In a sixth aspect of the invention, there is provided a method of modifying the glycosylation profile of a recombinant protein, the method comprising contacting a recombinant protein with either (i) the mammalian cell according to the first aspect of the invention; or (ii) at least one OST protein or functional fragment thereof, wherein the at least one OST protein is a *Trypanosoma spp.* OST protein, preferably wherein the at least one OST protein is a *Trypanosoma brucei* OST protein, more preferably wherein the at least one OST protein comprises an amino acid sequence according to any of SEQ ID NOs: 14, 15, 16, 17, 18, 19, and/or 20 or a sequence having at least 70% sequence identity thereto; or manufacturing the recombinant protein in a mammalian cell according to the first aspect of the invention, preferably wherein the mammalian cell is engineered to express the recombinant protein.

### BRIEF DESCRIPTION OF DRAWINGS

**Figure 1A** shows an amino acid sequence alignment of OST proteins (*Tb*STT3A, B and C) from *T. brucei*, residues 367-408, including the predicted active region 371-408. Positively charged residues are shaded in grey, negatively charged residues are in bold and underlined, and neutral charge residues are in bold.
**Figure 1B** shows the AlphaFold predicted structures of *Tb*STT3 A, B, C with the predicted active region highlighted in dark grey. Key amino acid residues are shown and labelled in black.
**Figure 2** shows a table representing the three OST enzymes isolated from *T*. *brucei* and their respective gene identification numbers.
**Figure 3** shows a table confirming the expression of *T. brucei* enzymes in mammalian cells, validated via mass spectrometry.
**Figure 4** demonstrates that hormone production increases in presence of kinetoplastid OST. Yield of EPO hormone detected 72h post transient transfection of cells. 'Mock' is mock transfected cell (control).
**Figure 5** demonstrates that N-glycosylation increases in presence of *T. brucei* OST. Data showing results of N glycan site occupancy in presence of OST (Enzyme A) and mock transfected cell (Control). Percentage of N glycosylated peptide was determined by Mass Spectrometry.
**Figure 6** demonstrates that transient expression of *T. brucei* OST enzymes in Expi293 cells, derived from HEK-293 cells, does not impact the overall viability of the cell population.

### DETAILED DESCRIPTION

In order that the present invention may be more readily understood, certain terms are first defined. Additional definitions are set forth throughout the detailed description.

As used herein, the term "mammalian cell" refers to a eukaryotic cell which has been derived or isolated from a tissue of a mammal. In some embodiments, the eukaryotic cell may be modified to proliferate indefinitely generating an immortalised cell line. In other embodiments, the eukaryotic cell may not be immortalised. In the context of the present invention, the mammalian cell is used as an expression system for the expression of a recombinant protein. As such, any mammalian cell which achieves this function is within the scope of the present invention. For example, the mammalian cell may be a Chinese hamster ovary (CHO) cell, a baby hamster kidney (BHK21) cell, a murine myeloma cell (NS0 and Sp2/0) or a human embryonic kidney (HEK) cell. Preferably, the mammalian cell of the present invention is a Chinese hamster ovary (CHO) cell, and/or an immortalized human embryonic kidney (HEK) cell, such as a HEK293 cell.

As used herein, the term "exogenous" refers to a nucleic acid sequence (i.e., a heterologous nucleic acid), also referred to as a sequence of DNA, that originated from outside the mammalian cell and is subsequently transformed into the mammalian cell. As will be understood by a person of skill in the art, the nucleic acid molecules of the present invention are exogenous to the mammalian cells of the present invention as they are derived from *Trypanosoma brucei* OST encoding nucleic acids. The term "nucleic acid sequence" and "gene" may be used interchangeably herein although it is understood that a "gene" is typically used to refer to a nucleic acid sequence or a combination of nucleic acid sequences that are transcribed and translated into a single protein, or multiple proteins that form a complex, with a specified function. In the context of the present invention, the term "exogenous gene" refers to a gene originating from a different species, specifically a parasite such as *Trypanosoma brucei,* and is therefore considered to be a heterologous gene. The exogenous nucleic acid sequences of the present invention may have a sequence according to any one of SEQ ID NOs: 1 to 7.

As used herein, the terms "oligosaccharyltransferase", "OST" and "OTase" are used interchangeably and can refer to single and multi-subunit glycosyltransferase enzymes that catalyses the addition of an N-glycan to a protein. In the context of the present invention, OSTs derived or obtained from parasites are used.

As used herein, the term "sequence identity" and "sequence homology" are interchangeable and refers to the number of identical residues over a defined length into a given alignment. To calculate % sequence identity of any of the sequences herein disclosed, sequence comparison software may be used, for example, using the default settings on the BLAST software package (V2.10.1).

As used herein the term "recombinant protein" refers to a protein that has been encoded by a particular nucleic acid sequence i.e., gene (recombinant DNA) that has been cloned in an expression system that supports expression of the gene and translation of the messenger RNA (mRNA). The gene introduced into the expression system may be a heterologous gene (or an exogenous gene), i.e., the gene originates or is derived from a cell type originating from a different organism to the recipient expression system. Methods for heterologous expression of recombinant proteins will be well known to those skilled in the art. The expression system of the present invention is a mammalian expression system. Preferably, the mammalian expression system is a mammalian cell, such as a CHO or HEK cell.

As used herein in relation to the methods of the invention, the term "contacting" is intended to include any means by which the recombinant protein and the at least one OST proteins are brought into contact sufficient to allow glycosylation of the recombinant protein by the at least one OST protein. This could involve chaperone proteins as required. It is envisaged that the recombinant protein may be contacted with the at least one OST protein either within a mammalian cell or in a cell-free system, for example a cell-free expression system. The recombinant protein and the at least one OST protein may be co-expressed in the mammalian cell or the cell-free system, or they may be expressed at different times. For example, the mammalian cell may constitutively express the at least one OST protein prior to expression of the recombinant cell by the mammalian cell. The at least one OST protein may be expressed and excreted from or extracted from the mammalian cell prior to contacting with the recombinant protein. For example, the mammalian cell expressing the at least one OST protein may be lysed to allow contact with the recombinant protein. As would be understood by a person of skill in the art, there are many ways in which the methods of the invention may be performed but the core concept requires that the at least one OST protein is presented to the recombinant protein in such a way as to allow for glycosylation of the recombinant protein. Ideally, the glycosylated recombinant protein is maintained in a conformation that maintains its biological activity as measured *in vitro* or *in vivo* by ligand affinity assays, activity assays, stability assays, half-life assays, pharmacokinetic assays, dosage studies, cell-uptake assays and other assays to assess potency, efficacy and quality. Again, a skilled person would know how to do this.

As used herein the term "glycosylation" refers to the process by which oligosaccharides (glycan moieties) are attached to a target molecule, such as proteins and lipids. The skilled person will readily recognise that this process is vital for both the functioning and stability of the protein. Oligosaccharides are carbohydrates consisting of chains or polymers of monosaccharides (single sugar molecules). Glycosylation is a form of co-translational and posttranslational modification of proteins in which carbohydrates are conjugated or covalently attached onto a protein. In biology, the process of glycosylation is an enzyme-catalysed reaction, and may involve enzymes such as oligosaccharyltransferase (OSTs) and glycosyltransferases. Within mammalian cells, glycosylation primarily occurs within the rough endoplasmic reticulum, cytoplasm, and nucleus. There are two major types of glycosylation: N-glycosylation (also known as "N-linked glycosylation") and O-glycosylation (also known as "O-linked glycosylation"), with the former being the most prevalent. There is also C-glycosylation (also known as "C-linked glycosylation") which occurs when a glycan is linked to a carbon atom on a tryptophan side chain. In N-glycosylation, glycans are attached to the nitrogen atoms of amino acids (for example, asparagine and arginine). In O-glycosylation, glycans are attached to the oxygen atoms of a hydroxyl group on amino acids (for examine, serine, threonine, and tyrosine).

As used herein the term "active region" refers to a region of the at least one OST protein which is proposed or predicted to interact with a target molecule, such as a polypeptide. Said interaction between the OST and target molecule may involve glycosylation and/or binding. For example, said interaction may involve OST-mediated glycosylation of a recombinant polypeptide or protein. The term "active region" may also be used interchangeably with "active site", "recognition site", "consensus", "conserved region" and "binding site" as used herein.

Accordingly, in the context of the present invention, the addition of "N-glycans" to a protein of interest is of particular interest. The addition of an N-glycan refers to the addition of any of the three general types of N-glycans: oligomannose, complex and hybrid, of which each contains the common core Man₃GlcNAc₂ with any number of branches and/or elongations.

As used herein the terms "N-glycan occupancy", "N-glycan site occupancy", N-glycosylation occupancy", and "N-glycosylation site occupancy" may be used interchangeably, and refer to the number of potential N-glycan (or N-glycosylation) sites on a protein that are occupied, i.e., the number of sequons modified. This is also known as the "macroheterogeneity" of N-glycosylation. For example, an N-glycan occupancy of 50% would reflect a protein with half of its potential N-glycan sites occupied. An N-glycan occupancy of 100% would reflect a protein having all of its potential N-glycan sites occupied. The present invention is particularly concerned with the N-glycan occupancy of NXS/T sequons (i.e., the number of NXS/T consensus sites which are occupied with a glycan).

As used herein, the term "N-glycosylation profile" refers to the site of N glycosylation and the type of N-glycan present at the site on the surface of the fully folded protein. The N-glycosylation profile therefore reflects both the degree of N-glycosylation site occupancy and the type of individual N-glycans on a fully folded protein. As would be appreciated by the skilled person, in determining the N-glycosylation profile, the degree of N-glycosylation site occupancy and type of N-glycans present will reflect the profile of all proteins in a given population of proteins (i.e., those proteins present in a sample). In particular, in the context of the present invention, the term "N-glycosylation profile" refers to the global profile of the degree of N-glycosylation site occupancy and type of N-glycans present in a given population of recombinant proteins. Preferably, in the context of the present invention, an improved glycosylation profile consists of an increase in the degree of N-glycosylation site occupancy on the recombinant protein with consistent N-glycan moieties that are reproducible between production batches of recombinant protein. Mass spectroscopy can be utilised in order to determine the % N-glycosylation occupancy of a given protein. Preferably, it is envisaged that the OST proteins of the present invention will increase the % N-glycosylation occupancy of a given protein, such as a recombinant protein, to at least 90% site occupancy. For example, at least 91%, at least 92%, at least 93%, at least 94%, at least 95%, at least 96%, at least 97%, at least 98%, at least 99%, or at least 100% N-glycan site occupancy.

As used herein, the terms "expression vector" or "expression construct" refer to a nucleic acid construct, generated recombinantly or synthetically, with a series of specified nucleic acid elements that permit transcription of a particular nucleic acid sequence in a host cell. The expression vector can be part of a plasmid, bacterial artificial chromosomes, virus, or nucleic acid sequence fragment. Typically, the expression vector includes a nucleic acid sequence linked to a promoter sequence. The expression vector may encode at least one of any of the nucleic acid sequences of SEQ ID NOs: 1, 2, 3, 4, 5, 6, 7, 8, 9, 10, 11, 12, 13, 21, and/or 22.

To produce the *T. brucei* OSTs in mammalian cells, the aforementioned expression vectors containing the nucleic acid sequence are used for stable transfection or transient transfection of the mammalian cell. A stable transfection refers to any method in which the *T. brucei* OST genes are integrated into the host genome of a mammalian cell that allows for stable expression of the OST genes under the control of a constitutive or inducible promoter. A stable transfection allows for integration into the host genome thus the gene is replicated, and the expression of the *T. brucei* OST genes are sustained long-term. A transient transfection refers to any method in which the *T. brucei* OST genes are introduced and expressed under the control of a constitutive or inducible promoter. A transient transfection does not allow for integration into the host genome thus the gene is not inherited during cell division, and the expression of the *T. brucei* OST genes is therefore for a finite period of time.

The use of the alternative (e.g., "or") should be understood to mean either one, both, or any combination thereof of the alternatives. As used herein, the indefinite articles "a" or "an" should be understood to refer to "one or more" of any recited or enumerated component.

As used herein, "about" means within an acceptable error range for the particular value as determined by one of ordinary skill in the art, which will depend in part on how the value is measured or determined, i.e., the limitations of the measurement system. For example, "about" can mean within 1 or more than 1 standard deviation per the practice in the art. Alternatively, "about" can mean a range of up to 20%. When particular values are provided in the application and claims, unless otherwise stated, the meaning of "about" should be assumed to be within an acceptable error range for that particular value.

It is envisaged that the expression system herein disclosed and methods thereof will have numerous benefits compared to an expression system that utilises native mammalian OSTs. For example, recombinant products produced in the expression system herein defined are expected to have improved stability and functionality, enhanced effectiveness, a higher yield and a higher batch-to-batch uniformity, all of which are key advantageous characteristics in the bioproduction industry.

In a first aspect, the present invention provides a mammalian cell comprising at least one nucleic acid sequence encoding at least one oligosaccharyltransferase (OST) protein or functional fragment thereof, wherein the at least one OST protein is a *Trypanosoma* spp. OST protein, preferably wherein the at least one OST protein is a *Trypanosoma brucei* OST protein.

In a second aspect, the present invention provides a mammalian cell comprising at least one nucleic acid sequence encoding at least one oligosaccharyltransferase (OST) protein or functional fragment thereof, wherein the at least one nucleic acid sequence encoding the at least one OST protein comprises a sequence according to any of SEQ ID NOs: 1, 2, 3, 4, 5, 6, 7, 8, 9, 10, 11, 12, 13, 21, and/or 22, or a sequence having at least 70% identity thereto. Accordingly, the present invention utilises genetic engineering of highly efficient OST enzymes from single-celled parasitic organisms into mammalian cells. As a result, the inventors envisage that an improvement in N-glycan occupancy as well as sialyation of recombinant proteins can be obtained. The nucleic acid sequence according to the first aspect may have a sequence according to any of SEQ ID NOs: 1, 2, 3, 4, 5, 6, 7, 8, 9, 10, 11, 12, 13, 21, and/or 22. Said sequences encode OST proteins from *Trypanosoma brucei (T. brucei),* the known causative agent of human African trypanosomiasis. There exist three subspecies which cause different types of trypanosomiasis: *T. brucei brucei, T. brucei gambiense, and T. brucei rhodiense.*

In any aspect or embodiment of the invention disclosed herein, it is envisaged that the cell or vector may include any combination of one, two, three or more of the nucleic acid sequences herein disclosed, particularly those combinations set out in the detailed description.

SEQ ID NO: 1 corresponds to a consensus DNA sequence encoding a fragment of the *Tb*STT3A protein.

SEQ ID NO: 21 corresponds to a consensus DNA sequence encoding a fragment of the *Tb*STT3B protein.

SEQ ID NO: 22 corresponds to a consensus DNA sequence encoding a fragment of the *Tb*STT3C protein.

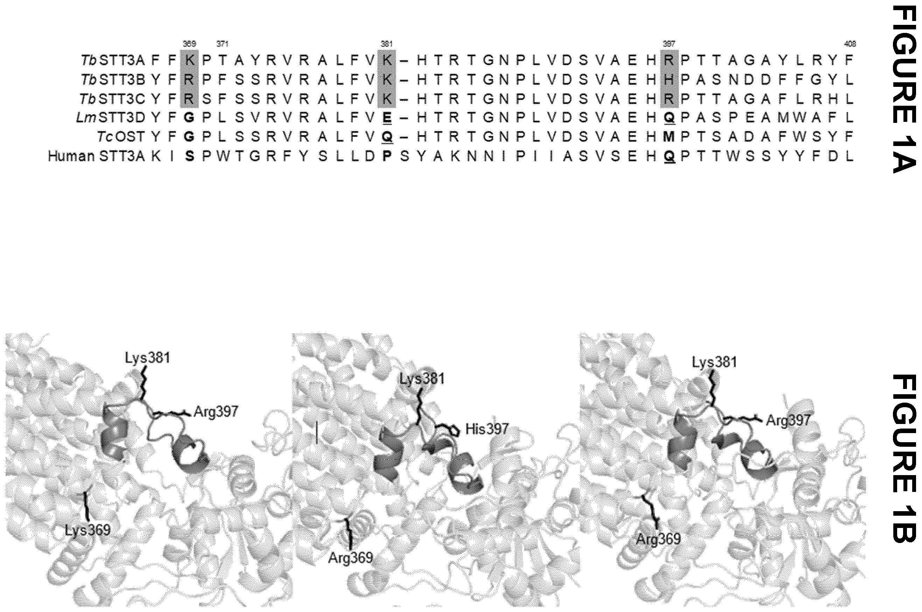

The highly conserved consensus sequence of SEQ ID NOs: 1, 21, and 22 may contain residues that are key to activity of the OST proteins. Without being bound by theory, it is envisaged that SEQ ID NOs: 1, 21, and 22 may encode the active region of the *Trypanosoma* OST proteins. This region defined by SEQ ID NOs: 1, 21, and 22 may be referred to as the active region, active site, recognition site, binding site, consensus site or highly conserved site herein. It is considered that the residues highlighted in Figure 1A may be particularly important. Therefore, the present invention also includes cells and vectors comprising any sequence with at least 70% identity to any of SEQ ID NOs: 1, 21, and/or 22 listed herein, yet including lysine-369, arginine-369, and/or lysine-381 as numbered according to their position in SEQ ID NOs: 8, 10, and 12.

SEQ ID NOs: 2, 4, and 6 correspond to a DNA sequence encoding the active region sequences of the *Tb*STT3A, *Tb*STT3B, and *Tb*STT3C proteins, respectively.

SEQ ID NOs: 3, 5, and 7 correspond to a DNA sequence encoding the active region sequences of the *Tb*STT3A, *Tb*STT3B, and *Tb*STT3C proteins, respectively, optimised for expression in CHO mammalian cells.

SEQ ID NOs: 8, 10, and 12 correspond to a DNA sequence encoding the full length sequences of the *Tb*STT3A, *Tb*STT3B, and *Tb*STT3C proteins, respectively.

SEQ ID NOs: 9, 11, and 13 correspond to a DNA sequence encoding the full length sequences of the *Tb*STT3A, *Tb*STT3B, and *Tb*STT3C proteins, respectively, optimised for expression in CHO mammalian cells. While SEQ ID NOs: 1-13 and 21-22 disclosed herein represent DNA nucleic acid sequences, the skilled person will appreciate that the corresponding RNA nucleic acid sequences would also be compatible with the disclosures of the present invention, as such the thymine residues may be replaced by uracil residues.

The active region of *T. brucei* OST enzymes is predicted to be within the region spanning amino acid residue numbers 371-408 of *Tb*STT3A, B and C. The inventors of the present invention have identified potentially key residues within and around this highly conserved region that may influence peptide specificity. These residues include lysine-369 or arginine-369 or lysine-381, and arginine-397 or histidine-397. In particular, the key amino acid residues in *Tb*STT3A are lysine-369, lysine-381, and arginine-397. The key amino acid residues in *Tb*STT3B are arginine-369, lysine-381, and histidine-397. The key amino acid residues in *Tb*STT3C are arginine-369, lysine-381, and arginine-397.

In accordance with a third aspect of the invention, there is provided a mammalian cell comprising at least one nucleic acid sequence encoding at least one oligosaccharyltransferase (OST) protein or functional fragment thereof, wherein the at least one OST protein comprises an amino acid sequence according to any of SEQ ID NOs: 14, 15, 16, 17, 18, 19, and/or 20 or a sequence having at least 70% sequence identity thereto. The OST protein may comprise the full length protein (i.e., comprise SEQ ID NOs: 17, 18, and/or 19), or the OST protein may comprise the highly conserved region (i.e., comprise SEQ ID NOs: 14, 15, 16, and/or 20). As would be readily understood by the skilled person, the amino acid sequences as defined in the third aspect of the invention may be derived from the nucleic acid sequences as defined in the first and second aspects of the invention. However, there are numerous permutations of combinations of nucleic acid codons which may produce these amino acid sequences, and this will also be understood by the skilled person. As such, there may be other nucleic acid sequences from which the amino acid sequences of the third aspect of the invention may be derivable.

SEQ ID NOs: 14, 15, and 16 correspond to an amino acid sequence corresponding to the active regions of the *Tb*STT3A, *Tb*STT3B, and *Tb*STT3C proteins, respectively.

SEQ ID NOs: 17, 18, and 19 correspond to an amino acid sequence corresponding to the full length *Tb*STT3A, *Tb*STT3B, and *Tb*STT3C proteins, respectively.

SEQ ID NO: 20 corresponds to amino acids that are conserved across the active region of *Tb*STT3A, *Tb*STT3B, and *Tb*STT3C. SEQ ID NOs: 1, 21, and 22 are consensus DNA sequences which encode an amino acid according to SEQ ID NO: 20.

It has been previously demonstrated by Jinnelov *et al.* (2017) that arginine-397 and histidine-397 in *T. brucei* OSTa (*Tb*STT3A) and *T. brucei* OSTb (*Tb*STT3B), respectively, are critical to determining peptide specificity between *T. brucei* OST enzymes. Of note, both of these residues are positively charged. The inventors of the present invention have observed that the residues that may be involved in peptide specificity at the active region of *L. major* and *T. cruzi* OST enzymes are neutral or negatively charged amino acids (i.e., that these key residues are not positively charged). Whereas, surprisingly, in contrast, the key residues that are likely to be involved in peptide specificity at the active region of *T. brucei* OST enzymes are positively charged. This pattern occurs two more times at the other residues mentioned above. Therefore, the inventors have hypothesized that the unique glycan specificity of the *T. brucei* OST proteins may be influenced by or be a result of the presence of positively charged residues at these positions.

The present invention also provides a nucleic acid comprising or having a sequence that has at least 70% identity to any one of SEQ ID NOs: 1, 2, 3, 4, 5, 6, 7, 8, 9, 10, 11, 12,13, 21, and/or 22. For example, the nucleic acid sequence may have at least 75% identity to SEQ ID NOs: 1, 2, 3, 4, 5, 6, 7, 8, 9, 10, 11, 12, 13, 21, and/or 22; at least 80% identity to SEQ ID NOs: 1, 2, 3, 4, 5, 6, 7, 8, 9, 10, 11, 12,13, 21, and/or 22; at least 85% identity to SEQ ID NOs: 1, 2, 3, 4, 5, 6, 7, 8, 9, 10, 11, 12, 13, 21, and/or 22; at least 90% identity to SEQ ID NOs: 1, 2, 3, 4, 5, 6, 7, 8, 9, 10, 11, 12,13, 21, and/or 22; at least 91% identity to SEQ ID NOs: 1, 2, 3, 4, 5, 6, 7, 8, 9, 10, 11, 12,13, 21, and/or 22; at least 92% identity to SEQ ID NOs: 1, 2, 3, 4, 5, 6, 7, 8, 9, 10, 11, 12,13, 21, and/or 22; at least 93% identity to SEQ ID NOs: 1, 2, 3, 4, 5, 6, 7, 8, 9, 10, 11, 12,13, 21, and/or 22; at least 94% identity to SEQ ID NOs: 1, 2, 3, 4, 5, 6, 7, 8, 9, 10, 11, 12, 13, 21, and/or 22; at least 95% identity to SEQ ID NOs: 1, 2, 3, 4, 5, 6, 7, 8, 9, 10, 11, 12,13, 21, and/or 22; at least 96% identity to SEQ ID NOs: 1, 2, 3, 4, 5, 6, 7, 8, 9, 10, 11, 12, 13, 21, and/or 22; at least 97% identity to SEQ ID NOs: 1, 2, 3, 4, 5, 6, 7, 8, 9, 10, 11, 12, 13, 21, and/or 22; at least 98% identity to SEQ ID NOs: 1, 2, 3, 4, 5, 6, 7, 8, 9, 10, 11, 12,13, 21, and/or 22; or at least 99% identity to SEQ ID NOs: 1, 2, 3, 4, 5, 6, 7, 8, 9, 10, 11, 12,13, 21, and/or 22.

The mammalian cell may comprise at least one exogenous nucleic acid comprising or having a sequence according to SEQ ID NO: 1. In a preferred embodiment, the exogenous nucleic acid has the sequence of SEQ ID NO: 1, or a sequence having at least 70% identity to said sequence. For example, the exogenous nucleic acid may comprise a sequence that has at least 75% identity to SEQ ID NO: 1; at least 80% identity to SEQ ID NO: 1; at least 85% identity to SEQ ID NO: 1; at least 90% identity to SEQ ID NO: 1; at least 91% identity to SEQ ID NO: 1; at least 92% identity to SEQ ID NO: 1; at least 93% identity to SEQ ID NO: 1; at least 94% identity to SEQ ID NO: 1; at least 95% identity to SEQ ID NO: 1; at least 96% identity to SEQ ID NO: 1; at least 97% identity to SEQ ID NO: 1; at least 98% identity to SEQ ID NO: 1; or at least 99% identity to SEQ ID NO: 1. In accordance with the sequence as shown in SEQ ID NO: 1, the amino acid residues at positions 369, 381 and 397 will be positively charged amino acids, such as amino acids selected from lysine, arginine and histidine, or artificial analogues thereof.

The mammalian cell may comprise at least one exogenous nucleic acid comprising or having a sequence according to SEQ ID NO: 21. In a preferred embodiment, the exogenous nucleic acid has the sequence of SEQ ID NO: 21, or a sequence having at least 70% identity to said sequence. For example, the exogenous nucleic acid may comprise a sequence that has at least 75% identity to SEQ ID NO: 21; at least 80% identity to SEQ ID NO: 21; at least 85% identity to SEQ ID NO: 21; at least 90% identity to SEQ ID NO: 21; at least 91% identity to SEQ ID NO: 21; at least 92% identity to SEQ ID NO: 21; at least 93% identity to SEQ ID NO: 21; at least 94% identity to SEQ ID NO: 21; at least 95% identity to SEQ ID NO: 21; at least 96% identity to SEQ ID NO: 21; at least 97% identity to SEQ ID NO: 21; at least 98% identity to SEQ ID NO: 21; or at least 99% identity to SEQ ID NO: 21. In accordance with the sequence as shown in SEQ ID NO: 21, the amino acid residues at positions 369, 381 and 397 will be positively charged amino acids, such as amino acids selected from lysine, arginine and histidine, or artificial analogues thereof.

The mammalian cell may comprise at least one exogenous nucleic acid comprising or having a sequence according to SEQ ID NO: 22. In a preferred embodiment, the exogenous nucleic acid has the sequence of SEQ ID NO: 22, or a sequence having at least 70% identity to said sequence. For example, the exogenous nucleic acid may comprise a sequence that has at least 75% identity to SEQ ID NO: 22; at least 80% identity to SEQ ID NO: 22; at least 85% identity to SEQ ID NO: 22; at least 90% identity to SEQ ID NO: 22; at least 91% identity to SEQ ID NO: 22; at least 92% identity to SEQ ID NO: 22; at least 93% identity to SEQ ID NO: 22; at least 94% identity to SEQ ID NO: 22; at least 95% identity to SEQ ID NO: 22; at least 96% identity to SEQ ID NO: 22; at least 97% identity to SEQ ID NO: 22; at least 98% identity to SEQ ID NO: 21; or at least 99% identity to SEQ ID NO: 22. In accordance with the sequence as shown in SEQ ID NO: 22, the amino acid residues at positions 369, 381 and 397 will be positively charged amino acids, such as amino acids selected from lysine, arginine and histidine, or artificial analogues thereof.

In any embodiment of the invention, SEQ ID NOs: 1, 21, and 22 are interchangeable.

The mammalian cell may comprise one or more exogenous nucleic acids encoding different OSTs, wherein the exogenous nucleic acid is selected from the group including any of SEQ ID NOs: 1, 2, 3, 4, 5, 6, 7, 8, 9, 10, 11, 12, 13, 21, and/or 22. The mammalian cell may comprise a single exogenous nucleic acid sequence, two exogenous nucleic acid sequences or three exogenous nucleic acid sequences. For example, the mammalian cell may comprise an exogenous nucleic acid sequence according to any one of SEQ ID NOs: 1, 2, 3, 4, 5, 6, 7, 8, 9, 10, 11, 12,13, 21, and/or 22; or any combination thereof. Where the mammalian cell comprises two or more exogenous nucleic acids, the exogenous nucleic acids may be expressed separately, i.e., on separate vectors, or together i.e., on the same vector. The one or more nucleic acid molecules may be expressed as fusion proteins or they may be expressed as separate proteins. They may be expressed as a fusion protein comprising a linker, such as a cleavable linker. The cleavable linker may be a liker that is cleavable by a protease or it may be a self-cleaving peptide, such as a viral self-cleaving peptide, such as P2A, as would be understood by a person of skill in the art.

In a preferred embodiment, the mammalian cell may comprise an exogenous nucleic acid sequence according to SEQ ID NOs: 2, 4, or 6; 2 and 4; 2 and 6; 4 and 6; and/or 2, 4 and 6. In another preferred embodiment, the mammalian cell may comprise an exogenous nucleic acid sequence according to SEQ ID NOs: 8, 10, or 12; 8 and 10; 8 and 12; 10 and 12; and/or 8, 10, and 12. In another preferred embodiment, the mammalian cell may comprise an exogenous nucleic acid sequence according to SEQ ID NOs: 3, 5, or 7; 3 and 5; 3 and 7; 5 and 7; and/or 3, 5 and 7. In another preferred embodiment, the mammalian cell may comprise an exogenous nucleic acid sequence according to SEQ ID NOs: 9, 11, or 13; 9 and 11; 9 and 13; 11 and 13; and/or 9, 11, and 13. As would be appreciated by the skilled person, any combination of the DNA sequences disclosed herein could be utilised for the purposes of the present invention, including combinations of optimised nucleic acid sequences with non-optimised nucleic acid sequences. Further, the skilled person will readily understand that any of the nucleic acid sequences (optimised or non-optimised) encoding an active region disclosed herein may be combined with any of the nucleic acid sequences (optimised or non-optimised) encoding a full length protein disclosed herein.

In a preferred embodiment, the exogenous nucleic acid has a sequence of SEQ ID NO: 2, or a sequence having at least 70% identity to said sequence. For example, the exogenous nucleic acid may comprise a sequence that has at least 75% identity to SEQ ID NO: 2; at least 80% identity to SEQ ID NO: 2; at least 85% identity to SEQ ID NO: 2; at least 90% identity to SEQ ID NO: 2; at least 91% identity to SEQ ID NO: 2; at least 92% identity to SEQ ID NO: 2; at least 93% identity to SEQ ID NO: 2; at least 94% identity to SEQ ID NO: 2; at least 95% identity to SEQ ID NO: 2; at least 96% identity to SEQ ID NO: 2; at least 97% identity to SEQ ID NO: 2; at least 98% identity to SEQ ID NO: 2; or at least 99% identity to SEQ ID NO: 2.

In a preferred embodiment, the exogenous nucleic acid has a sequence of SEQ ID NO: 3, or a sequence having at least 70% identity to said sequence. For example, the exogenous nucleic acid may comprise a sequence that has at least 75% identity to SEQ ID NO: 3; at least 80% identity to SEQ ID NO: 3; at least 85% identity to SEQ ID NO: 3; at least 90% identity to SEQ ID NO: 3; at least 91% identity to SEQ ID NO: 3; at least 93% identity to SEQ ID NO: 3; at least 93% identity to SEQ ID NO: 3; at least 94% identity to SEQ ID NO: 3; at least 95% identity to SEQ ID NO: 3; at least 96% identity to SEQ ID NO: 3; at least 97% identity to SEQ ID NO: 3; at least 98% identity to SEQ ID NO: 3; or at least 99% identity to SEQ ID NO: 3.

In a preferred embodiment, the exogenous nucleic acid has a sequence of SEQ ID NO: 4, or a sequence having at least 70% identity to said sequence. For example, the exogenous nucleic acid may comprise a sequence that has at least 75% identity to SEQ ID NO: 4; at least 80% identity to SEQ ID NO: 4; at least 85% identity to SEQ ID NO: 4; at least 90% identity to SEQ ID NO: 4; at least 91% identity to SEQ ID NO: 4; at least 94% identity to SEQ ID NO: 4; at least 94% identity to SEQ ID NO: 4; at least 94% identity to SEQ ID NO: 4; at least 95% identity to SEQ ID NO: 4; at least 96% identity to SEQ ID NO: 4; at least 97% identity to SEQ ID NO: 4; at least 98% identity to SEQ ID NO: 4; or at least 99% identity to SEQ ID NO: 4.

In a preferred embodiment, the exogenous nucleic acid has a sequence of SEQ ID NO: 5, or a sequence having at least 70% identity to said sequence. For example, the exogenous nucleic acid may comprise a sequence that has at least 75% identity to SEQ ID NO: 5; at least 80% identity to SEQ ID NO: 5; at least 85% identity to SEQ ID NO: 5; at least 90% identity to SEQ ID NO: 5; at least 91% identity to SEQ ID NO: 5; at least 92% identity to SEQ ID NO: 5; at least 93% identity to SEQ ID NO: 5; at least 94% identity to SEQ ID NO: 5; at least 95% identity to SEQ ID NO: 5; at least 96% identity to SEQ ID NO: 5; at least 97% identity to SEQ ID NO: 5; at least 98% identity to SEQ ID NO: 5; or at least 99% identity to SEQ ID NO: 5. It is envisaged that this particular exogenous nucleic acid may be particularly successful in improving N-glycan occupancy due to its broad substrate specificity and expression.

In a preferred embodiment, the exogenous nucleic acid has a sequence of SEQ ID NO: 6, or a sequence having at least 70% identity to said sequence. For example, the exogenous nucleic acid may comprise a sequence that has at least 75% identity to SEQ ID NO: 6; at least 80% identity to SEQ ID NO: 6; at least 85% identity to SEQ ID NO: 6; at least 90% identity to SEQ ID NO: 6; at least 91% identity to SEQ ID NO: 6; at least 92% identity to SEQ ID NO: 6; at least 93% identity to SEQ ID NO: 6; at least 94% identity to SEQ ID NO: 6; at least 95% identity to SEQ ID NO: 6; at least 96% identity to SEQ ID NO: 6; at least 97% identity to SEQ ID NO: 6; at least 98% identity to SEQ ID NO: 6; or at least 99% identity to SEQ ID NO: 6.

In a preferred embodiment, the exogenous nucleic acid has a sequence of SEQ ID NO: 7, or a sequence having at least 70% identity to said sequence. For example, the exogenous nucleic acid may comprise a sequence that has at least 75% identity to SEQ ID NO: 7; at least 80% identity to SEQ ID NO: 7; at least 85% identity to SEQ ID NO: 7; at least 90% identity to SEQ ID NO: 7; at least 91% identity to SEQ ID NO: 7; at least 92% identity to SEQ ID NO: 7; at least 93% identity to SEQ ID NO: 7; at least 94% identity to SEQ ID NO: 7; at least 95% identity to SEQ ID NO: 7; at least 96% identity to SEQ ID NO: 7; at least 97% identity to SEQ ID NO: 7; at least 98% identity to SEQ ID NO: 7; or at least 99% identity to SEQ ID NO: 7.

In a preferred embodiment, the exogenous nucleic acid has a sequence of SEQ ID NO: 8, or a sequence having at least 70% identity to said sequence. For example, the exogenous nucleic acid may comprise a sequence that has at least 75% identity to SEQ ID NO: 8; at least 80% identity to SEQ ID NO: 8; at least 85% identity to SEQ ID NO: 8; at least 90% identity to SEQ ID NO: 8; at least 91% identity to SEQ ID NO: 8; at least 92% identity to SEQ ID NO: 8; at least 93% identity to SEQ ID NO: 8; at least 94% identity to SEQ ID NO: 8; at least 95% identity to SEQ ID NO: 8; at least 96% identity to SEQ ID NO: 8; at least 97% identity to SEQ ID NO: 8; at least 98% identity to SEQ ID NO: 8; or at least 99% identity to SEQ ID NO: 8.

In a preferred embodiment, the exogenous nucleic acid has a sequence of SEQ ID NO: 9, or a sequence having at least 70% identity to said sequence. For example, the exogenous nucleic acid may comprise a sequence that has at least 75% identity to SEQ ID NO: 9; at least 80% identity to SEQ ID NO: 9; at least 85% identity to SEQ ID NO: 9; at least 90% identity to SEQ ID NO: 9; at least 91% identity to SEQ ID NO: 9; at least 92% identity to SEQ ID NO: 9; at least 93% identity to SEQ ID NO: 9; at least 94% identity to SEQ ID NO: 9; at least 95% identity to SEQ ID NO: 9; at least 96% identity to SEQ ID NO: 9; at least 97% identity to SEQ ID NO: 9; at least 98% identity to SEQ ID NO: 9; or at least 99% identity to SEQ ID NO: 9.

In a preferred embodiment, the exogenous nucleic acid has a sequence of SEQ ID NO: 10, or a sequence having at least 70% identity to said sequence. For example, the exogenous nucleic acid may comprise a sequence that has at least 75% identity to SEQ ID NO: 10; at least 80% identity to SEQ ID NO: 10; at least 85% identity to SEQ ID NO: 10; at least 90% identity to SEQ ID NO: 10; at least 91% identity to SEQ ID NO: 10; at least 92% identity to SEQ ID NO: 10; at least 93% identity to SEQ ID NO: 10; at least 94% identity to SEQ ID NO: 10; at least 95% identity to SEQ ID NO: 10; at least 96% identity to SEQ ID NO: 10; at least 97% identity to SEQ ID NO: 10; at least 98% identity to SEQ ID NO: 10; or at least 99% identity to SEQ ID NO: 10.

In a preferred embodiment, the exogenous nucleic acid has a sequence of SEQ ID NO: 11, or a sequence having at least 70% identity to said sequence. For example, the exogenous nucleic acid may comprise a sequence that has at least 75% identity to SEQ ID NO: 11; at least 80% identity to SEQ ID NO: 11; at least 85% identity to SEQ ID NO: 11; at least 90% identity to SEQ ID NO: 11; at least 91% identity to SEQ ID NO: 11; at least 92% identity to SEQ ID NO: 11; at least 93% identity to SEQ ID NO: 11; at least 94% identity to SEQ ID NO: 11; at least 95% identity to SEQ ID NO: 11; at least 96% identity to SEQ ID NO: 11; at least 97% identity to SEQ ID NO: 11; at least 98% identity to SEQ ID NO: 11; or at least 99% identity to SEQ ID NO: 11.

In a preferred embodiment, the exogenous nucleic acid has a sequence of SEQ ID NO: 12, or a sequence having at least 70% identity to said sequence. For example, the exogenous nucleic acid may comprise a sequence that has at least 75% identity to SEQ ID NO: 12; at least 80% identity to SEQ ID NO: 12; at least 85% identity to SEQ ID NO: 12; at least 90% identity to SEQ ID NO: 12; at least 91% identity to SEQ ID NO: 12; at least 92% identity to SEQ ID NO: 12; at least 93% identity to SEQ ID NO: 12; at least 94% identity to SEQ ID NO: 12; at least 95% identity to SEQ ID NO: 12; at least 96% identity to SEQ ID NO: 12; at least 97% identity to SEQ ID NO: 12; at least 98% identity to SEQ ID NO: 12; or at least 99% identity to SEQ ID NO: 12.

In a preferred embodiment, the exogenous nucleic acid has a sequence of SEQ ID NO: 13, or a sequence having at least 70% identity to said sequence. For example, the exogenous nucleic acid may comprise a sequence that has at least 75% identity to SEQ ID NO: 13; at least 80% identity to SEQ ID NO: 13; at least 85% identity to SEQ ID NO: 13; at least 90% identity to SEQ ID NO: 13; at least 91% identity to SEQ ID NO: 13; at least 92% identity to SEQ ID NO: 13; at least 93% identity to SEQ ID NO: 13; at least 94% identity to SEQ ID NO: 13; at least 95% identity to SEQ ID NO: 13; at least 96% identity to SEQ ID NO: 13; at least 97% identity to SEQ ID NO: 13; at least 98% identity to SEQ ID NO: 13; or at least 99% identity to SEQ ID NO: 13.

The three OST paralogs expressed in *T. brucei* have distinct substrate specificities. Analysis of parasite protein glycosylation showed that *Tb*STT3A selectively transfers biantennary Man₃GlcNAc₂ glycans whereas both *Tb*STT3B and *Tb*STT3C transfer triantennary Man₉GlcNAc₂ glycans. It has been suggested that substrate specificity is derived from the presence of the *ALG12-*dependent c-branch of the conventional triantennary Man₉GlcNAc₂, which is required by *Tb*STT3B and *Tb*STT3C but not *Tb*STT3A. However, studies also reported that *both Tb*STT3A and *Tb*STT3B can transfer Man₅GlcNAc₂ glycans as well as Man₇GlcNAc₂ glycans to a *T*. *brucei* VSG protein. The unique functionality of *Tb*STT3B in reference to its promiscuous substrate specificity is of pertinence. Most OTases display a distinct preference for an oligosaccharide, thereby limiting the efficiency of N-glycosylation. Indeed, harnessing an adaptable OTase that can unbiasedly transfer N-glycans to almost any amino acid sequence would conceivably increase N-glycosylation in mammalian cells. Therefore, it is desirable that in an alternative embodiment of the present invention, the *T. brucei* OST enzymes are provided in combinations which utilise their unique specificities and functionalities.

Accordingly, in an alternative preferred embodiment, the mammalian cell may comprise two exogenous nucleic acids encoding between them at least the active regions of *Tb*STT3B and *Tb*STT3C. For example, the exogenous nucleic acid may comprise a sequence according to SEQ ID NO: 4, or a sequence having at least 70% identity to said sequence, and a further sequence according to SEQ ID NO: 6, or a sequence having at least 70% identity to said sequence. For example, the exogenous nucleic acid may comprise a sequence that has at least 75% identity to SEQ ID NO: 4 and/or 6; at least 80% identity to SEQ ID NO: 4 and/or 6; at least 85% identity to SEQ ID NO: 4 and/or 6; at least 90% identity to SEQ ID NO: 4 and/or 6; at least 91% identity to SEQ ID NO: 4 and/or 6; at least 92% identity to SEQ ID NO: 4 and/or 6; at least 93% identity to SEQ ID NO: 4 and/or 6; at least 94% identity to SEQ ID NO: 4 and/or 6; at least 95% identity to SEQ ID NO: 4 and/or 6; at least 96% identity to SEQ ID NO: 4 and/or 6; at least 97% identity to SEQ ID NO: 4 and/or 6; at least 98% identity to SEQ ID NO: 4 and/or 6; or at least 99% identity to SEQ ID NO: 4 and/or 6.

In an alternative preferred embodiment, the mammalian cell may comprise two exogenous nucleic acids encoding full length *Tb*STT3B and *Tb*STT3C. For example, the exogenous nucleic acid may comprise a sequence according to SEQ ID NO: 10, or a sequence having at least 70% identity to said sequence, and a further sequence according to SEQ ID NO: 12, or a sequence having at least 70% identity to said sequence. For example, the exogenous nucleic acid may comprise a sequence that has at least 75% identity to SEQ ID NO: 10 and/or 12; at least 80% identity to SEQ ID NO: 10 and/or 12; at least 85% identity to SEQ ID NO: 10 and/or 12; at least 90% identity to SEQ ID NO: 10 and/or 12; at least 91% identity to SEQ ID NO: 10 and/or 12; at least 92% identity to SEQ ID NO: 10 and/or 12; at least 93% identity to SEQ ID NO: 10 and/or 12; at least 94% identity to SEQ ID NO: 10 and/or 12; at least 95% identity to SEQ ID NO: 10 and/or 12; at least 96% identity to SEQ ID NO: 10 and/or 12; at least 97% identity to SEQ ID NO: 10 and/or 12; at least 98% identity to SEQ ID NO: 10 and/or 12; or at least 99% identity to SEQ ID NO: 10 and/or 12.

In an alternative preferred embodiment, the mammalian cell may comprise two exogenous nucleic acids encoding at least the active regions of *Tb*STT3A and *Tb*STT3B. For example, the exogenous nucleic acid may comprise a sequence according to SEQ ID NO: 2, or a sequence having at least 70% identity to said sequence, and a further sequence according to SEQ ID NO: 4, or a sequence having at least 70% identity to said sequence. For example, the exogenous nucleic acid may comprise a sequence that has at least 75% identity to SEQ ID NO: 2 and/or 4; at least 80% identity to SEQ ID NO: 2 and/or 4; at least 85% identity to SEQ ID NO: 2 and/or 4; at least 90% identity to SEQ ID NO: 2 and/or 4; at least 91% identity to SEQ ID NO: 2 and/or 4; at least 92% identity to SEQ ID NO: 2 and/or 4; at least 93% identity to SEQ ID NO: 2 and/or 4; at least 94% identity to SEQ ID NO: 2 and/or 4; at least 95% identity to SEQ ID NO: 2 and/or 4; at least 96% identity to SEQ ID NO: 2 and/or 4; at least 97% identity to SEQ ID NO: 2 and/or 4; at least 98% identity to SEQ ID NO: 2 and/or 4; or at least 99% identity to SEQ ID NO: 2 and/or 4.

In an alternative preferred embodiment, the mammalian cell may comprise two exogenous nucleic acids encoding the full length *Tb*STT3A and *Tb*STT3B. For example, the mammalian cell may comprise two exogenous nucleic acids. The exogenous nucleic acid may comprise a sequence according to SEQ ID NO: 8, or a sequence having at least 70% identity to said sequence, and a further sequence according to SEQ ID NO: 10, or a sequence having at least 70% identity to said sequence. For example, the exogenous nucleic acid may comprise a sequence that has at least 75% identity to SEQ ID NO: 8 and/or 10; at least 80% identity to SEQ ID NO: 8 and/or 10; at least 85% identity to SEQ ID NO: 8 and/or 10; at least 90% identity to SEQ ID NO: 8 and/or 10; at least 91% identity to SEQ ID NO: 8 and/or 10; at least 92% identity to SEQ ID NO: 8 and/or 10; at least 93% identity to SEQ ID NO: 8 and/or 10; at least 94% identity to SEQ ID NO: 8 and/or 10; at least 95% identity to SEQ ID NO: 8 and/or 10; at least 96% identity to SEQ ID NO: 8 and/or 10; at least 97% identity to SEQ ID NO: 8 and/or 10; at least 98% identity to SEQ ID NO: 8 and/or 10; or at least 99% identity to SEQ ID NO: 8 and/or 10.

In an alternative preferred embodiment, the mammalian cell may comprise two exogenous nucleic acids encoding at least the active regions of *Tb*STT3A and *Tb*STT3C. For example, the mammalian cell may comprise two exogenous nucleic acids. The exogenous nucleic acid may comprise a sequence according to SEQ ID NO: 2, or a sequence having at least 70% identity to said sequence, and a further sequence according to SEQ ID NO: 6, or a sequence having at least 70% identity to said sequence. For example, the exogenous nucleic acid may comprise a sequence that has at least 75% identity to SEQ ID NO: 2 and/or 6; at least 80% identity to SEQ ID NO: 2 and/or 6; at least 85% identity to SEQ ID NO: 2 and/or 6; at least 90% identity to SEQ ID NO: 2 and/or 6; at least 91% identity to SEQ ID NO: 2 and/or 6; at least 92% identity to SEQ ID NO: 2 and/or 6; at least 93% identity to SEQ ID NO: 2 and/or 6; at least 94% identity to SEQ ID NO: 2 and/or 6; at least 95% identity to SEQ ID NO: 2 and/or 6; at least 96% identity to SEQ ID NO: 2 and/or 6; at least 97% identity to SEQ ID NO: 2 and/or 6; at least 98% identity to SEQ ID NO: 2 and/or 6; or at least 99% identity to SEQ ID NO: 2 and/or 6.

In an alternative preferred embodiment, the mammalian cell may comprise two exogenous nucleic acids encoding the full length *Tb*STT3A and *Tb*STT3C. For example, the exogenous nucleic acid may comprise a sequence according to SEQ ID NO: 8, or a sequence having at least 70% identity to said sequence, and a further sequence according to SEQ ID NO: 12, or a sequence having at least 70% identity to said sequence. For example, the exogenous nucleic acid may comprise a sequence that has at least 75% identity to SEQ ID NO: 8 and/or 12; at least 80% identity to SEQ ID NO: 8 and/or 12; at least 85% identity to SEQ ID NO: 8 and/or 12; at least 90% identity to SEQ ID NO: 8 and/or 12; at least 91% identity to SEQ ID NO: 8 and/or 12; at least 92% identity to SEQ ID NO: 8 and/or 12; at least 93% identity to SEQ ID NO: 8 and/or 12; at least 94% identity to SEQ ID NO: 8 and/or 12; at least 95% identity to SEQ ID NO: 8 and/or 12; at least 96% identity to SEQ ID NO: 8 and/or 12; at least 97% identity to SEQ ID NO: 8 and/or 12; at least 98% identity to SEQ ID NO: 8 and/or 12; or at least 99% identity to SEQ ID NO: 8 and/or 12.

In a more preferred embodiment, the mammalian cell may comprise three exogenous nucleic acids encoding at least the active regions of *Tb*STT3A, *Tb*STT3B, and *Tb*STT3C. For example, the exogenous nucleic acid may comprise a sequence according to SEQ ID NO: 2, or a sequence having at least 70% identity to said sequence, a further sequence according to SEQ ID NO: 4, or a sequence having at least 70% identity to said sequence, and a sequence according to SEQ ID NO: 6, or a sequence having at least 70% identity to said sequence. For example, the exogenous nucleic acid may comprise a sequence that has at least 75% identity to SEQ ID NO: 2, 4 and/or 6; at least 80% identity to SEQ ID NO: 2, 4 and/or 6; at least 85% identity to SEQ ID NO: 2, 4 and/or 6; at least 90% identity to SEQ ID NO: 2, 4 and/or 6; at least 91% identity to SEQ ID NO: 2, 4 and/or 6; at least 92% identity to SEQ ID NO: 2, 4 and/or 6; at least 93% identity to SEQ ID NO: 2, 4 and/or 6; at least 94% identity to SEQ ID NO: 2, 4 and/or 6; at least 95% identity to SEQ ID NO: 2, 4 and/or 6; at least 96% identity to SEQ ID NO: 2, 4 and/or 6; at least 97% identity to SEQ ID NO: 2, 4 and/or 6; at least 98% identity to SEQ ID NO: 2, 4 and/or 6; or at least 99% identity to SEQ ID NO: 2, 4 and/or 6.

In an alternative preferred embodiment, the mammalian cell may comprise three exogenous nucleic acids encoding the full length *Tb*STT3A, *Tb*STT3B, and *Tb*STT3C. For example, the exogenous nucleic acid may comprise a sequence according to SEQ ID NO: 8, or a sequence having at least 70% identity to said sequence, a further sequence according to SEQ ID NO: 10, or a sequence having at least 70% identity to said sequence, and a further sequence according to SEQ ID NO: 12, or a sequence having at least 70% identity to said sequence. For example, the exogenous nucleic acid may comprise a sequence that has at least 75% identity to SEQ ID NO: 8, 10 and/or 12; at least 80% identity to SEQ ID NO: 8, 10 and/or 12; at least 85% identity to SEQ ID NO: 8, 10 and/or 12; at least 90% identity to SEQ ID NO: 5 and/or 3; at least 91% identity to SEQ ID NO: 8, 10 and/or 12; at least 92% identity to SEQ ID NO: 8, 10 and/or 12; at least 93% identity to SEQ ID NO: 8, 10 and/or 12; at least 94% identity to SEQ ID NO: 8, 10 and/or 12; at least 95% identity to SEQ ID NO: 8, 10 and/or 12; at least 96% identity to SEQ ID NO: 8, 10 and/or 12; at least 97% identity to SEQ ID NO: 8, 10 and/or 12; at least 98% identity to SEQ ID NO: 8, 10 and/or 12; or at least 99% identity to SEQ ID NO: 8, 10 and/or 12.

The skilled person will readily understand that any of the abovementioned combinations can also be used with the optimised versions of the sequences disclosed herein. For example, the mammalian cell may comprise two optimised exogenous nucleic acids encoding at least the active regions of any of *Tb*STT3A, *Tb*STT3B and/or *Tb*STT3C, thus comprising or having a sequence according to SEQ ID NOs: 5 and/or 7; 3 and/or 5; 3 and/or 7; or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto. Alternatively, the mammalian cell may comprise three optimised exogenous nucleic acids encoding the active regions of any *Tb*STT3A, *Tb*STT3B and *Tb*STT3C, thus comprising or having a sequence according to SEQ ID NOs: 3, 5, and/or 7; or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto. Similarly, the mammalian cell may comprise two optimised exogenous nucleic acids encoding the full length of any of *Tb*STT3A, *Tb*STT3B and/or *Tb*STT3C, thus comprising or having a sequence according to SEQ ID NOs: 11 and/or 13; 9 and/or 11; 9 and/or 13; or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto. Alternatively, the mammalian cell may comprise three optimised exogenous nucleic acids encoding the full length of any *Tb*STT3A, *Tb*STT3B and *Tb*STT3C, thus comprising or having a sequence according to SEQ ID NOs: 9, 11 and/or 13; or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto. The skilled person will also readily understand that any combination of the DNA sequences disclosed herein could be utilised for the purposes of the present invention, including combinations of optimised nucleic acid sequences with non-optimised nucleic acid sequences.

Further, the skilled person will readily understand that any of the nucleic acid sequences (optimised or non-optimised) encoding an active region disclosed herein may be combined with any of the nucleic acid sequences (optimised or non-optimised) encoding a full length protein disclosed herein. For example, where there are two exogenous nucleic acids, the nucleic acid sequence may comprise any of the sequences according to SEQ ID NOs: 2, 3, 8 and/or 9 or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto, and further comprise any of the sequences according to SEQ ID NOs: 4, 5, 10 and/or 11, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto.

Further, the nucleic acid sequences may comprise any of the sequences according to SEQ ID NOs: 2, 3, 8 and/or 9 or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto, and further comprise any of the sequences according to SEQ ID NOs: 6, 7, 12 and/or 13, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto.

Further, the nucleic acid sequences may comprise any of the sequences according to SEQ ID NOs: 4, 5, 10 and/or 11, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto, and further comprise any of the sequences according to SEQ ID NOs: 6, 7, 12 and/or 13, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto.

Where there are three exogenous nucleic acids, the nucleic acid sequence may comprise any of the sequences according to SEQ ID NOs: 2, 3, 8 and/or 9 or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto, and further comprise any of the sequences according to SEQ ID NOs: 4, 5, 10 and/or 11, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto, and further comprise any of the sequences according to SEQ ID NOs: 6, 7, 12 and/or 13, or sequences having at least 70%, at least 75%, at least 80%, at least 85%, at least 90%, at least 91%, at least 92%, at least 93%, at least 94%, at least 95%, at least 96%, at least 97%, at least 98%, or at least 99% sequence identity thereto.

In a fourth aspect, the present invention provides an isolated nucleic acid molecule comprising a sequence according to any of SEQ ID NOs: 1, 2, 3, 4, 5, 6, 7, 8, 9, 10, 11, 12, 13, 21 and/or 22, or a sequence having at least 70% identity to thereto, preferably wherein the isolated nucleic acid molecule comprises a sequence according to SEQ ID NOs: 3, 5, 7, 9, 11, and/or 13. As such, in a preferred embodiment, the isolated nucleic acid molecule comprises a sequence according to SEQ ID NOs: 3, 5, 7, 9, 11, and/or 13 (i.e., a sequence optimised for expression in mammalian cells such as CHO cells). It is envisaged that the nucleic acid of the fourth aspect of the invention may be successfully transfected into mammalian cells. In another embodiment, the nucleic acid comprises a sequence according to SEQ ID NOs: 2, 4, 6, 8, 10, and/or 12 (i.e., a sequence which has not been optimised for expression in mammalian cells such as CHO cells). Again, the skilled person will readily understand that any combination of the nucleic acid sequences disclosed herein could be utilised for the purposes of the present invention, including combinations of optimised nucleic acid sequences with non-optimised nucleic acid sequences. Further, the skilled person will readily understand that any of the nucleic acid sequences (optimised or non-optimised) encoding a active region disclosed herein may be combined with any of the nucleic acid sequences (optimised or non-optimised) encoding a full length protein disclosed herein.

Accordingly, in a fifth aspect, the present invention provides a vector comprising at least one nucleic acid sequence according to SEQ ID NOs: 1, 2, 3, 4, 5, 6, 7, 8, 9, 10, 11, 12, 13, 21 and/or 22, or comprising the isolated nucleic acid molecule of the fourth aspect of the invention. It is envisaged that a suitable vector may be designed to encode the at least one nucleic acid comprising or having a sequence according to SEQ ID NOs: 1, 2, 3, 4, 5, 6, 7, 8, 9, 10, 11, 12, 13, 21, and/or 22 for transfection into mammalian cells. In one embodiment, the vector comprises at least one nucleic acid sequence according to SEQ ID NOs: 3, 5, 7, 9, 11 and/or 13 (i.e., a sequence optimised for expression in mammalian cells such as CHO cells). It is envisaged that the vector of the fourth aspect of the invention may be successfully transfected into mammalian cells. In another embodiment, the vector comprises at least one nucleic acid sequence according to SEQ ID NOs: 2, 4, 6, 8, 10 and/or 12 (i.e., a sequence which has not been optimised for expression in mammalian cells such as CHO cells). Again, the skilled person will readily understand that any combination of the DNA sequences disclosed herein could be utilised for the purposes of the present invention, including combinations of optimised nucleic acid sequences with non-optimised nucleic acid sequences.

Suitable vectors may include, but are not limited to plasmid-based expression vectors, bacterial artificial chromosome (BAC) vectors, or viral vectors (e.g., adenovirus, retrovirus, lentivirus). The methods for transfection into mammalian cells are conventional in the art and will be readily understood by the skilled person. For example, suitable transfection methods may include, but are not limited to, electroporation, viral transfection, microinjection, and chemical transfection (e.g., reagent-based transfection).

The mammalian cell of the present invention may be any mammalian cell in which the OSTs from the parasites herein disclosed can be successfully transfected and expressed. For example, the mammalian cell may be a Chinese hamster ovary (CHO) cell, a baby hamster kidney (BHK21) cell, a murine myeloma cell (such as NS0 and Sp2/0), or a human embryonic kidney (such as HEK293) cell. In a preferred embodiment, the mammalian cell is a CHO cell or HEK293 cell.

Both transient and stable mammalian cell lines may be used in the present invention. Preferably, the mammalian cell to be used is a stable mammalian cell. Even more preferably, the mammalian cell to be used is a stable CHO cell. Stable mammalian cells have an advantage over transiently transfected mammalian cells in that they allow for the genetic modification to be passed on to subsequent generations of cells, allowing for cells that have been genetically modified to maintain stable expression of a gene of interest over extended periods of time.

The present invention discloses a mammalian cell comprising at least one exogenous nucleic acid sequence encoding at least one OST from a *T. brucei.* The exogenous nucleic acid sequence may be expressed by the mammalian cell to provide a functional protein. Expression of the nucleic acid sequence in mammalian cells may be achieved via inducible expression, constitutive expression, stable expression, and transient expression.

In one embodiment, the mammalian cell may express the at least one *T. brucei* OST enzyme and one or more recombinant proteins encoded by a further exogenous nucleic acid sequence. The recombinant protein will be a protein of interest that is to be glycosylated by the *T. brucei* OST enzyme. As would be understood by the skilled person, the recombinant protein could be any protein or peptide comprising amino acid residues amenable to N-glycosylation. For example, the recombinant protein could be a therapeutic protein or therapeutic peptide, a diagnostic protein or diagnostic peptide, a protein or peptide for research and development purposes and/or a protein or peptide for supplementing growth media, which requires or benefits from N-glycosylation in mammalian cells in order to improve its yield, activity, stability, and/or reproducibility.

The recombinant protein may be any protein that can be produced using the mammalian expression system herein disclosed. A key advantage of the present invention is that its application is not limited to a small number of recombinant proteins, but can be applied to any recombinant protein of interest. For example, the recombinant protein may be a therapeutic protein. Examples of therapeutic proteins include, but are not limited to, a hormone, a cytokine, an antibody, an enzyme, a complement protein, a blood clotting factor, a functional fragment thereof, or any combination thereof. The antibody may be a monoclonal or polyclonal antibody. The antibody may be of origin from the following classes and subclasses: IgG including IgG1, IgG2, IgG3 and IgG4 subclasses, IgA including lgA1 and IgA2 subclasses, IgM, IgD and IgE. The antibody may be a humanised monoclonal antibody. The antibody may be a bispecific antibody.

The term "functional fragment thereof", as used in relation to OST proteins in any embodiment of the invention or in relation to the recombinant protein or any other protein referred to herein, refers to a polypeptide which is derived from a longer polypeptide e.g., the full length polypeptide, and which has been truncated in the N-terminal region and/or the C-terminal region to generate a fragment of said full length polypeptide. To be a functional fragment, the fragment must maintain at least 40%, preferably at least 50%, more preferably at least 60%, more preferably at least 70%, more preferably at least 80%, even more preferably at least 90%, most preferably at least 95%, and even most preferably at least 100% of the activity of the full-length/mature polypeptide.

In a preferred embodiment, the recombinant protein may be a protein selected from the group comprising erythropoietin (EPO), Retuximab, butyrylcholinesterase (BuChE), Factor VIII, ENPP1-Fc, Olamkicept (sgp130-Fc), GM-CSF, FSH, eCG, alpha-1-antitrypsin, viral glycoproteins, for example, SARS-CoV2 spike protein, or any combination thereof.

In an alternative embodiment, the mammalian cell may express the at least one *T. brucei* OST enzyme alone without the recombinant protein. It is envisaged that such cells could be further modified to express any recombinant protein or proteins of interest that require N-glycosylation by the *T. brucei* OST enzymes. Additionally, said mammalian cell may be further modified to become increasingly amenable to the introduction of a further exogenous nucleic acid sequence encoding the recombinant protein.

In a preferred embodiment, the mammalian cell expresses the OST protein to provide a functional/active OST protein. Specifically, the mammalian cell may produce at least one OST protein comprising or having an amino acid sequence according to any one of SEQ ID NOs: 14, 15, 16, 17, 18, 19 and/or 20, or a sequence having at least 70% identity to said sequence.

The mammalian cells may produce at least one OST protein comprising or having an amino acid sequence according to any one of SEQ ID NOs: 14, 15, and/or 16, or a functional fragment thereof, or a sequence having at least 70% identity to said sequence or fragment. Alternatively, the mammalian cells may produce at least one OST protein comprising or having an amino acid sequence according to any one of SEQ ID NOs: 17, 18, and/or 19, or a functional fragment thereof, or a sequence having at least 70% identity to said sequence or fragment.

The mammalian cell may produce at least one OST protein comprising or having an amino acid sequence according to any one of SEQ ID NOs: 14, 15, 16, 17, 18, 19 and/or 20, or a functional fragment thereof, or any combination of such sequence or fragment. The mammalian cell may comprise a single OST protein, two OST proteins or three OST proteins.

Accordingly, in one embodiment, the mammalian cell may comprise one OST protein. In a preferred embodiment, the OST protein has a sequence of SEQ ID NO: 14, or a sequence having at least 70% identity to said sequence. For example the OST protein may have a sequence that has at least 75% identity to SEQ ID NO: 14; at least 80% identity to SEQ ID NO: 14; at least 85% identity to SEQ ID NO: 14; at least 90% identity to SEQ ID NO: 14; at least 91% identity to SEQ ID NO: 14; at least 92% identity to SEQ ID NO: 14; at least 93% identity to SEQ ID NO: 14; at least 94% identity to SEQ ID NO: 14; at least 95% identity to SEQ ID NO: 14; at least 96% identity to SEQ ID NO: 14; at least 97% identity to SEQ ID NO: 14; at least 98% identity to SEQ ID NO: 14; or at least 99% identity to SEQ ID NO: 14.

In a preferred embodiment, the OST protein has a sequence of SEQ ID NO: 15, or a sequence having at least 70% identity to said sequence. For example the OST protein may have a sequence that has at least 75% identity to SEQ ID NO: 15; at least 80% identity to SEQ ID NO: 15; at least 85% identity to SEQ ID NO: 15; at least 90% identity to SEQ ID NO: 15; at least 91% identity to SEQ ID NO: 15; at least 92% identity to SEQ ID NO: 15; at least 93% identity to SEQ ID NO: 15; at least 94% identity to SEQ ID NO: 15; at least 95% identity to SEQ ID NO: 15; at least 96% identity to SEQ ID NO: 15; at least 97% identity to SEQ ID NO: 15; at least 98% identity to SEQ ID NO: 15; or at least 99% identity to SEQ ID NO: 15.

In a preferred embodiment, the OST protein has a sequence of SEQ ID NO: 16, or a sequence having at least 70% identity to said sequence. For example the OST protein may have a sequence that has at least 75% identity to SEQ ID NO: 16; at least 80% identity to SEQ ID NO: 16; at least 85% identity to SEQ ID NO: 16; at least 90% identity to SEQ ID NO: 16; at least 91% identity to SEQ ID NO: 16; at least 92% identity to SEQ ID NO: 16; at least 93% identity to SEQ ID NO: 16; at least 94% identity to SEQ ID NO: 16; at least 95% identity to SEQ ID NO: 16; at least 96% identity to SEQ ID NO: 16; at least 97% identity to SEQ ID NO: 16; at least 98% identity to SEQ ID NO: 16; or at least 99% identity to SEQ ID NO: 16.

In a preferred embodiment, the OST protein has a sequence of SEQ ID NO: 17, or a sequence having at least 70% identity to said sequence. For example the OST protein may have a sequence that has at least 75% identity to SEQ ID NO: 17; at least 80% identity to SEQ ID NO: 17; at least 85% identity to SEQ ID NO: 17; at least 90% identity to SEQ ID NO: 17; at least 91% identity to SEQ ID NO: 17; at least 92% identity to SEQ ID NO: 17; at least 93% identity to SEQ ID NO: 17; at least 94% identity to SEQ ID NO: 17; at least 95% identity to SEQ ID NO: 17; at least 96% identity to SEQ ID NO: 17; at least 97% identity to SEQ ID NO: 17; at least 98% identity to SEQ ID NO: 17; or at least 99% identity to SEQ ID NO: 17.

In a preferred embodiment, the OST protein has a sequence of SEQ ID NO: 18, or a sequence having at least 70% identity to said sequence. For example the OST protein may have a sequence that has at least 75% identity to SEQ ID NO: 18; at least 80% identity to SEQ ID NO: 18; at least 85% identity to SEQ ID NO: 18; at least 90% identity to SEQ ID NO: 18; at least 91% identity to SEQ ID NO: 18; at least 92% identity to SEQ ID NO: 18; at least 93% identity to SEQ ID NO: 18; at least 94% identity to SEQ ID NO: 18; at least 95% identity to SEQ ID NO: 18; at least 96% identity to SEQ ID NO: 18; at least 97% identity to SEQ ID NO: 18; at least 98% identity to SEQ ID NO: 18; or at least 99% identity to SEQ ID NO: 18.

In a preferred embodiment, the OST protein has a sequence of SEQ ID NO: 19, or a sequence having at least 70% identity to said sequence. For example the OST protein may have a sequence that has at least 75% identity to SEQ ID NO: 19; at least 80% identity to SEQ ID NO: 19; at least 85% identity to SEQ ID NO: 19; at least 90% identity to SEQ ID NO: 19; at least 91% identity to SEQ ID NO: 19; at least 92% identity to SEQ ID NO: 19; at least 93% identity to SEQ ID NO: 19; at least 94% identity to SEQ ID NO: 19; at least 95% identity to SEQ ID NO: 19; at least 96% identity to SEQ ID NO: 19; at least 97% identity to SEQ ID NO: 19; at least 98% identity to SEQ ID NO: 19; or at least 99% identity to SEQ ID NO: 19.

In a preferred embodiment, the OST protein has a sequence of SEQ ID NO: 20, or a sequence having at least 70% identity to said sequence. For example the OST protein may have a sequence that has at least 75% identity to SEQ ID NO: 20; at least 80% identity to SEQ ID NO: 20; at least 85% identity to SEQ ID NO: 20; at least 90% identity to SEQ ID NO: 20; at least 91% identity to SEQ ID NO: 20; at least 92% identity to SEQ ID NO: 20; at least 93% identity to SEQ ID NO: 20; at least 94% identity to SEQ ID NO: 20; at least 95% identity to SEQ ID NO: 20; at least 96% identity to SEQ ID NO: 20; at least 97% identity to SEQ ID NO: 20; at least 98% identity to SEQ ID NO: 20; or at least 99% identity to SEQ ID NO: 20.

The mammalian cell may produce at least one OST protein comprising or having an amino acid sequence according to any one of SEQ ID NOs: 14, 15, 16, 17, 18, 19 and/or 20 or any combination thereof. The mammalian cell may comprise a single OST protein, two OST proteins or three OST proteins.

Accordingly, in another embodiment, the mammalian cell may comprise or express two separate OST proteins. For example, the OST proteins may have a sequence comprising the active region of *Tb*STT3B and *Tb*STT3C, thus comprising or having a sequence according to SEQ ID NO: 15, or a sequence having at least 70% identity to said sequence, and a further sequence according to SEQ ID NO: 16, or a sequence having at least 70% identity to said sequence. For example, the OST protein may have a sequence that has at least 75% identity to SEQ ID NO: 15 and/or 16; at least 80% identity to SEQ ID NO: 15 and/or 16; at least 85% identity to SEQ ID NO: 15 and/or 16; at least 90% identity to SEQ ID NO: 15 and/or 16; at least 91% identity to SEQ ID NO: 15 and/or 16; at least 92% identity to SEQ ID NO: 15 and/or 16; at least 93% identity to SEQ ID NO: 15 and/or 16; at least 94% identity to SEQ ID NO: 15 and/or 16; at least 95% identity to SEQ ID NO: 15 and/or 16; at least 96% identity to SEQ ID NO: 15 and/or 16; at least 97% identity to SEQ ID NO: 15 and/or 16; at least 98% identity to SEQ ID NO: 15 and/or 16; or at least 99% identity to SEQ ID NO: 15 and/or 16.

In another embodiment, the OST protein may have sequences comprising the full length *Tb*STT3B and *Tb*STT3C, thus comprising or having a sequence according to SEQ ID NO: 18, or a sequence having at least 70% identity to said sequence, and a further sequence according to SEQ ID NO: 19, or a sequence having at least 70% identity to said sequence. For example, the OST protein may have a sequence that has at least 75% identity to SEQ ID NO: 18 and/or 19; at least 80% identity to SEQ ID NO: 18 and/or 19; at least 85% identity to SEQ ID NO: 18 and/or 19; at least 90% identity to SEQ ID NO: 18 and/or 19; at least 91% identity to SEQ ID NO: 18 and/or 19; at least 92% identity to SEQ ID NO: 18 and/or 19; at least 93% identity to SEQ ID NO: 18 and/or 19; at least 94% identity to SEQ ID NO: 18 and/or 19; at least 95% identity to SEQ ID NO: 18 and/or 19; at least 96% identity to SEQ ID NO: 18 and/or 19; at least 97% identity to SEQ ID NO: 18 and/or 19; at least 98% identity to SEQ ID NO: 18 and/or 19; or at least 99% identity to SEQ ID NO: 18 and/or 19.

In another embodiment, the OST protein may have sequences comprising the active region of *Tb*STT3A and *Tb*STT3B, thus comprising or having a sequence according to SEQ ID NO: 14, or a sequence having at least 70% identity to said sequence, and a further sequence according to SEQ ID NO: 15, or a sequence having at least 70% identity to said sequence. For example, the OST protein may have a sequence that has at least 75% identity to SEQ ID NO: 14 and/or 15; at least 80% identity to SEQ ID NO: 14 and/or 15; at least 85% identity to SEQ ID NO: 14 and/or 15; at least 90% identity to SEQ ID NO: 14 and/or 15; at least 91% identity to SEQ ID NO: 14 and/or 15; at least 92% identity to SEQ ID NO: 14 and/or 15; at least 93% identity to SEQ ID NO: 14 and/or 15; at least 94% identity to SEQ ID NO: 14 and/or 15; at least 95% identity to SEQ ID NO: 14 and/or 15; at least 96% identity to SEQ ID NO: 14 and/or 15; at least 97% identity to SEQ ID NO: 14 and/or 15; at least 98% identity to SEQ ID NO: 14 and/or 15; or at least 99% identity to SEQ ID NO: 14 and/or 15.

In another embodiment, the OST protein may have sequences comprising the full length *Tb*STT3A and *Tb*STT3B, thus comprising or having a sequence according to SEQ ID NO: 17, or a sequence having at least 70% identity to said sequence, and a further sequence according to SEQ ID NO: 18, or a sequence having at least 70% identity to said sequence. For example, the OST protein may have a sequence that has at least 75% identity to SEQ ID NO: 17 and/or 18; at least 80% identity to SEQ ID NO: 17 and/or 18; at least 85% identity to SEQ ID NO: 17 and/or 18; at least 90% identity to SEQ ID NO: 17 and/or 18; at least 91% identity to SEQ ID NO: 17 and/or 18; at least 92% identity to SEQ ID NO: 17 and/or 18; at least 93% identity to SEQ ID NO: 17 and/or 18; at least 94% identity to SEQ ID NO: 17 and/or 18; at least 95% identity to SEQ ID NO: 17 and/or 18; at least 96% identity to SEQ ID NO: 17 and/or 18; at least 97% identity to SEQ ID NO: 17 and/or 18; at least 98% identity to SEQ ID NO: 17 and/or 18; or at least 99% identity to SEQ ID NO: 17 and/or 18.

In another embodiment, the OST protein may have sequences comprising the active region of *Tb*STT3A and *Tb*STT3C, thus comprising or having a sequence according to SEQ ID NO: 14, or a sequence having at least 70% identity to said sequence, and a further sequence according to SEQ ID NO: 16, or a sequence having at least 70% identity to said sequence. For example, the OST protein may have a sequence that has at least 75% identity to SEQ ID NO: 14 and/or 16; at least 80% identity to SEQ ID NO: 14 and/or 16; at least 85% identity to SEQ ID NO: 14 and/or 16; at least 90% identity to SEQ ID NO: 14 and/or 16; at least 91% identity to SEQ ID NO: 14 and/or 16; at least 92% identity to SEQ ID NO: 14 and/or 16; at least 93% identity to SEQ ID NO: 14 and/or 16; at least 94% identity to SEQ ID NO: 14 and/or 16; at least 95% identity to SEQ ID NO: 14 and/or 16; at least 96% identity to SEQ ID NO: 14 and/or 16; at least 97% identity to SEQ ID NO: 14 and/or 16; at least 98% identity to SEQ ID NO: 14 and/or 16; or at least 99% identity to SEQ ID NO: 14 and/or 16.

In another embodiment, the OST protein may have sequences comprising the full length *Tb*STT3A and *Tb*STT3C, thus comprising or having a sequence according to SEQ ID NO: 17, or a sequence having at least 70% identity to said sequence, and a further sequence according to SEQ ID NO: 19, or a sequence having at least 70% identity to said sequence. For example, the OST protein may have a sequence that has at least 75% identity to SEQ ID NO: 17 and/or 19; at least 80% identity to SEQ ID NO: 17 and/or 19; at least 85% identity to SEQ ID NO: 17 and/or 19; at least 90% identity to SEQ ID NO: 17 and/or 19; at least 91% identity to SEQ ID NO: 17 and/or 19; at least 92% identity to SEQ ID NO: 17 and/or 19; at least 93% identity to SEQ ID NO: 17 and/or 19; at least 94% identity to SEQ ID NO: 17 and/or 19; at least 95% identity to SEQ ID NO: 17 and/or 19; at least 96% identity to SEQ ID NO: 17 and/or 19; at least 97% identity to SEQ ID NO: 17 and/or 19; at least 98% identity to SEQ ID NO: 17 and/or 19; or at least 99% identity to SEQ ID NO: 17 and/or 19.

For the avoidance of any doubt, it is envisaged that when the cell comprises nucleic acids encoding more than one OST protein according to the disclosure, that these sequences are not necessarily on the same nucleic acid molecule or construct. Currently, nucleic acids encoding OST proteins are on separate nucleic acid molecules or genetic constructs that are used for transient and stable expression of the OST proteins. Nevertheless, such possibilities of having nucleic acids encoding more than one OST protein on the same nucleic acid molecule are not excluded by the disclosure herein, which also encompasses co-expression of the multiple OST molecules as fusion proteins or proteins separated by self-cleaving peptide sequences (e.g. viral P2A sequences) or enzymatically cleavable sequences, such as sequences susceptible to protease-mediated degradation or multiple expression cassettes within the same vector.

The skilled person will readily understand that any of the amino acid sequences comprising an active region may be combined with any of the amino acid sequences corresponding to a full length protein.

The mammalian cell may produce an OST protein comprising or having an amino acid sequence according to any one of SEQ ID NOs: 14, 15, 16, 17, 18, 19 and/or 20, or any combination thereof. The mammalian cell may comprise a single OST protein, two OST proteins or three OST proteins.

Accordingly, in one embodiment, the mammalian cell may comprise three separate OST proteins. For example, the OST protein may have a sequence comprising the proposed polypeptide binding site of *Tb*STT3A, *Tb*STT3B and *Tb*STT3C, thus comprising or having a sequence according to SEQ ID NO: 14, or a sequence having at least 70% identity to said sequence, and a further sequence according to SEQ ID NO: 15, or a sequence having at least 70% identity to said sequence, and a further sequence according to SEQ ID NO: 16, or a sequence having at least 70% identity to said sequence. For example, the OST protein may have a sequence that has at least 75% identity to SEQ ID NO: 14, 15 and/or 16; at least 80% identity to SEQ ID NO: 14, 15 and/or 16; at least 85% identity to SEQ ID NO: 14, 15 and/or 16; at least 90% identity to SEQ ID NO: 14, 15 and/or 16; at least 91% identity to SEQ ID NO: 14, 15 and/or 16; at least 92% identity to SEQ ID NO: 14, 15 and/or 16; at least 93% identity to SEQ ID NO: 14, 15 and/or 16; at least 94% identity to SEQ ID NO: 14, 15 and/or 16; at least 95% identity to SEQ ID NO: 14, 15 and/or 16; at least 96% identity to SEQ ID NO: 14, 15 and/or 16; at least 97% identity to SEQ ID NO: 14, 15 and/or 16; at least 98% identity to SEQ ID NO: 14, 15 and/or 16; or at least 99% identity to SEQ ID NO: 14, 15 and/or 16.

In another embodiment, the OST protein may have a sequence comprising the full length *Tb*STT3A, *Tb*STT3B and *Tb*STT3C, thus comprising or having a sequence according to SEQ ID NO: 17, or a sequence having at least 70% identity to said sequence, and a further sequence according to SEQ ID NO: 18, or a sequence having at least 70% identity to said sequence, and a further sequence according to SEQ ID NO: 19, or a sequence having at least 70% identity to said sequence. For example, the OST protein may have a sequence that has at least 75% identity to SEQ ID NO: 17, 18 and/or 19; at least 80% identity to SEQ ID NO: 17, 18 and/or 19; at least 85% identity to SEQ ID NO: 17, 18 and/or 19; at least 90% identity to SEQ ID NO: 17, 18 and/or 19; at least 91% identity to SEQ ID NO: 17, 18 and/or 19; at least 92% identity to SEQ ID NO: 17, 18 and/or 19; at least 93% identity to SEQ ID NO: 17, 18 and/or 19; at least 94% identity to SEQ ID NO: 17, 18 and/or 19; at least 95% identity to SEQ ID NO: 17, 18 and/or 19; at least 96% identity to SEQ ID NO: 17, 18 and/or 19; at least 97% identity to SEQ ID NO: 17, 18 and/or 19; at least 98% identity to SEQ ID NO: 17, 18 and/or 19; or at least 99% identity to SEQ ID NO: 17, 18 and/or 19.

The skilled person will readily understand that any of the amino acid sequences comprising an proposed polypeptide binding site (highly conserved region) may be combined with any of the amino acid sequences corresponding to a full length protein.

The native gene sequences from *T. brucei* encoding for OST enzymes have been codon-optimised for recombinant protein production in CHO cells. As will be readily understood by the skilled person, codon optimization is a gene-engineering tool that is performed to improve gene expression and protein production. Codon optimization attempts to address the issue of codon bias, which refers to synonymous codons that are used more often than other synonymous codons within regions of transcribed genes. The frequency of these synonymous codons varies within and among species. Codon optimization alters the gene sequence to accommodate the codon bias of the host organism without altering the amino acid sequence in order to increase protein production. The GenScript codon optimization tool was used. Accordingly, while the present invention discloses codon-optimised sequences for recombinant protein production in CHO cells, the skilled person would appreciate that the same could be done for recombinant protein production in other mammalian cells, including HEK cells (such as HEK293 cells).

In accordance with SEQ ID NOs: 3, 5, 7, 9, 11, and/or 13, disclosed herein, the inventors of the present invention have developed nucleic acid sequences encoding for *T. brucei* OST proteins (according to SEQ ID NOs: 14, 15, 16, 17, 8, and/or 19) in which the codons have been optimised for production in mammalian cells.

In a sixth aspect, the present invention provides a method of modifying the glycosylation profile of a recombinant protein, the method comprising contacting a recombinant protein with either (i) the mammalian cell according to the first aspect of the invention; or (ii) an OST protein or functional fragment thereof, wherein the OST protein is a *Trypanosoma spp.* OST protein, preferably wherein the OST protein is a *Trypanosoma brucei* OST protein, more preferably wherein the OST protein comprises an amino acid sequence according to any of SEQ ID NOs: 14, 15, 16, 17, 18, 19 and/or 20; or manufacturing the recombinant protein in a mammalian cell according to the first aspect of the invention, preferably wherein the mammalian cell is engineered to express the recombinant protein.

*Tb*STT3 proteins target certain proteins via recognition of a consensus region on the target by the *Tb*STT3 active region. *Tb*STT3 proteins have polypeptide specificity for certain acceptor substrates, and the active region involved in peptide acceptor specificity by *Tb*STT3s is predicted to fall within the region spanning amino acid positions 371-408 for each of *Tb*STT3A, *Tb*STT3B and *Tb*STT3C (Figure 1). In particular, arginine-397 in *Tb*STT3A and *Tb*STT3C and histidine-397 in *Tb*STT3B is known to interact with NX(S/T)-adjacent amino acids and influence interactions between the acceptor peptide and the enzyme surface (Jinnelov *et al*., 2017). These interactions can increase the efficiency of substrate recognition and thus this region is important for the specific function of *Tb*STT3 proteins. The residues arginine-397/histidine-397 in TbSTT3s are both positively charged amino acids. Potentially, without wishing to be bound by theory, the positively charged active region helps to define *Tb*STT3 function by promoting specific sequence preferences or polypeptide acceptor specificities. Thus, this unique property of *Tb*STT3 proteins could be particularly useful for biotechnology platforms to boost efficient N-glycosylation of recombinant glycoproteins in eukaryotic expression systems.

The three STT3 paralogs in *T. brucei* have distinct substrate specificities. For example, *Tb*STT3A has been found to selectively transfer biantennary Man₅GlcNAc₂ glycans, whereas both *Tb*STT3B and *Tb*STT3C transfer triantennary Man₉GlcNAc₂ glycans (Jinnelov *et al.*, 2017). Of particular importance is the ability of *Tb*STT3 proteins to transfer the Man₉GlcNAc₂ moiety, which is the preferred substrate for mammalian OST proteins.

The present invention discloses the genetic engineering of highly efficient enzymes from single celled parasitic organisms *(T. brucei)* into mammalian cell lines to boost N-glycan occupancy of recombinant proteins. Accordingly, the method herein disclosed provides a method in which the frequency of N-glycan occupancy on the recombinant protein may be altered. In a preferred embodiment, the method herein disclosed provides a method in which the frequency of N-glycan occupancy on the recombinant protein may be increased. However, there can also be an increase in sialyation and density of glycans on the surface of a recombinant protein. The term "increased" in the context of the present invention refers to the comparison between the number of potential N-glycosylation sites (i.e., N-glycan occupancy) on the recombinant protein produced in mammalian cell lines with OSTs in their native/unmodified form and the N-glycan occupancy on the recombinant protein produced in mammalian cell lines with OSTs obtained from parasites herein disclosed.

As disclosed above, the recombinant protein involved in the method of modifying the glycosylation profile of a recombinant protein may be any protein that can be produced using the mammalian expression system herein disclosed. For example, the recombinant protein may be a therapeutic protein. Examples of therapeutic proteins include, but are not limited to, a hormone, a cytokine, an antibody, an enzyme, a complement protein, a blood clotting factor, a functional fragment thereof, or any combination thereof. The antibody may be a monoclonal or polyclonal antibody. The antibody may be a humanised antibody. The antibody may be a bispecific antibody. The term "functional fragment thereof" refers to a polypeptide which is derived from a longer polypeptide e.g., the full length polypeptide, and which has been truncated in the N-terminal region and/or the C-terminal region to generate a fragment of said full length polypeptide. To be a functional fragment, the fragment must maintain at least 40%, preferably at least 50%, more preferably at least 60%, more preferably at least 70%, more preferably at least 80%, even more preferably at least 90%, most preferably at least 95%, and even most preferably at least 100% of the activity of the full-length/mature polypeptide.

In a preferred embodiment, the recombinant protein may be a protein selected from the group comprising erythropoietin (EPO), Retuximab, butyrylcholinesterase (BuChE), Factor VIII, ENPP1-Fc, Olamkicept (sgp130-Fc), GM-CSF, FSH, eCG, alpha-1-antitrypsin, viral glycoproteins, for example, SARS-CoV2 spike protein, or any combination thereof.

The invention is further described with reference to the following non-limiting examples:

### EXAMPLES

### Expression of Trypanosoma OSTs in mammalian cells results in improved yield and N-glycan site occupancy of therapeutic targets

### Example 1: Results

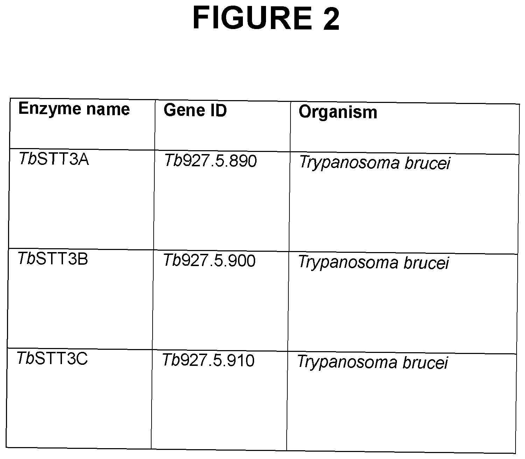

We sought to study the effects of expressing OSTs, originating from *T. brucei*, in mammalian cells used for therapeutic bioproduction (Figure 2). For this, studies were carried out using a previously validated suspension-adapted CHO cell line that was engineered to stably express the recombinant hormone erythropoietin (CHO-EPO). Four OST enzymes were selected that originate from *T. brucei.* Each OST was cloned into a mammalian expression vector used for transient expression. The OST vectors were transfected into the CHO-EPO cell line and OST and EPO expression was monitored. As a control, a mock transfection using an empty mammalian expression vector was performed on the CHO-EPO cell line.

### T. brucei OSTs are expressed in mammalian cells

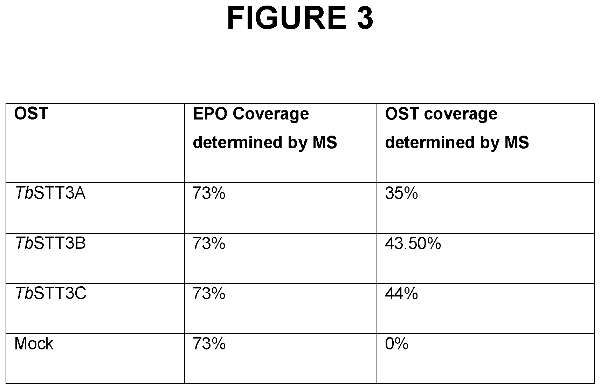

Following transfection, the cell number, and the viability, clumping, and diameter of the cells were assessed. To confirm expression of the relevant OSTs, a cell pellet of each transiently transfected cell line was taken 72h post-transfection and sent for proteomic analysis by mass spectrometry (MS) to identify presence of the OST. In all transiently transfected cell lines, the relevant OST was detected by MS, as well as the stably expressed EPO (Figure 3).

### T. brucei OSTs increase yield of therapeutic protein secreted by CHO cells

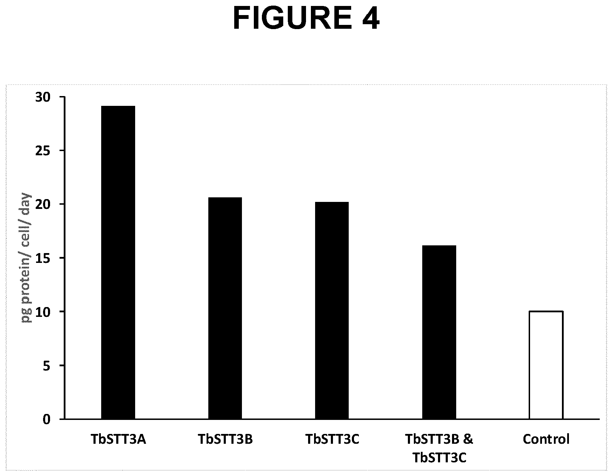

To examine the impact of OST expression on the yield of therapeutic proteins produced by cells, total protein yield in the supernatant was measured after 3 days. This showed an increase in EPO production relative to the mock transfected cell line upon introduction of each of the OSTs (Figure 4). For example, in the case of enzyme A (*Tb*STT3A), we observed a 2.75-fold increase in recombinant EPO production, demonstrating the predicted and intended effect of the heterologous OST enzymes on improving protein secretion.

### T. brucei OSTs increase N-glycan site occupancy of EPO produced in CHO cells

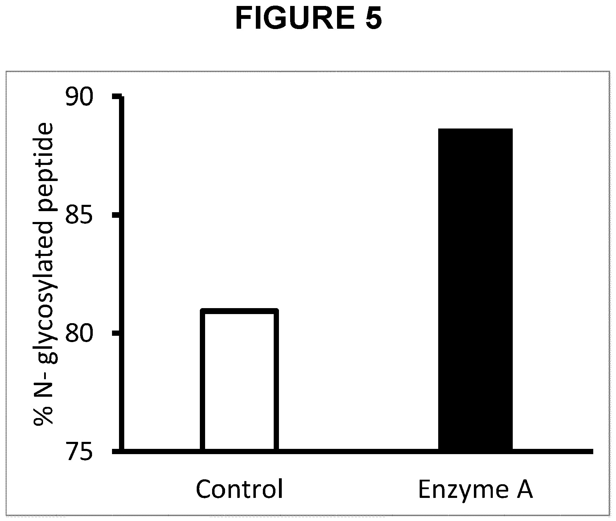

We next determined whether heterolougous OST expression changed N-glycosylation levels of secreted therapeutic proteins. For this, EPO was purified from OST-transfected cells and digested with PNGaseF for N-glycan site occupancy analysis. The number of N-glycan sites occupied on the EPO-derived peptides increased from 80% occupied to 89% occupied (Figure 5), demonstrating that OST expression can modify the N-glycosylation state of therapeutic proteins, even when only transiently expressed over a short period of time.

### Concluding remarks

This study demonstrates that OSTs originating from *T. brucei* can indeed be expressed in mammalian cells, and that their presence increases both yield and modifications on therapeutic proteins produced in the cell. This study focused exclusively on EPO hormone production, similar findings are expected for production of other therapeutic proteins in mammalian cells.

### Example 2: Methods

### Transfections

Cells were transfected according to standard protocol. In brief, cells were washed in electroporation buffer and a total of 160 ug of plasmid DNA was mixed with 40,000,000 CHO cells, making a final volume of 400 uL in electroporation buffer. Electroporation was carried out according to manufacturer's instructions, and immediately after transfection, the 400 uL cells were transferred to a 125 mL shake flask and placed in a CO2-incubator at 37C. After 40 minutes 30 mL of growth medium was added and the shake flask. Protein concentration in the supernatant was then measured.

### Purification

For whole proteome analysis, cell pellets were harvested. EPO was purified with an EPO purification kit.

### Mass spectrometry analysis

### Whole proteome

### S-Trap processing of samples

Cell pellets were lysed in lysis buffer (100 mM TEAB, 5% SDS), sonicated three times to shear DNA and protein concentration estimated using a micro-BCA assay. Aliquots of 350 µg proteins were processed using S-Trap mini protocol. Protein disulphide bonds were reduced in the presence of 20 mM DTT, then alkylated in 40 mM IAA. After sample application into S-Trap mini spin column, 5 washes with S-Trap binding buffer were performed. Peptides were digested with trypsin (1:40) overnight at 37°C and tryptic peptides were pooled, dried and quantified.

### Mass Spectrometry analysis

Peptides (equivalent of 1.5 µg) were injected onto a nanoscale C18 reverse-phase chromatography system and electrosprayed into an Orbitrap Mass Spectrometer. Peptides were eluted from the column at a constant flow rate and two blanks were run between each sample to reduce carry-over. The column was kept at a constant temperature of 50°C. The MS was operated in DIA mode.

### N-glycan site occupancy studies on purified EPO

### PNGase Treatment

Purified EPO samples were mixed with 4 µl of PNGase buffer and dried in a speed vac. Following this 20µl of heavy water was added to each sample and the mixture dried again. Dried PNGase F was resuspended in 10 µl heavy water, vortexed for 3 seconds and the content added to the samples, another 10 µl heavy water were added to the PNGase vial, vortexed for 2 seconds and the content added to the samples. The mixture (20µl) was then incubated at 50°C for 20min.

### Peptide digestion

Samples were reduced using DTT, and alkylated by adding 5 µl lodoacetamide (final concentration 300 mM). Samples and markers were then run on a bis-tris 4-12% gradient gel, washed, stained with Coomassie stain and protein bands cut out and gel pieces were washed.

In-gel digestion was carried out with trypsin at a final concentration of 12pg/mL (in 20 mM ammonium bicarbonate) and incubation at 30°C for 16 hr on a shaker. Acetonitrile (equal volume used to cover gel pieces) was added and peptide mixtures were extracted by shaking at 30°C for 15 min. The resulting supernatant was transferred to a fresh microcentrifuge tube. Peptides in the gel pieces were further extracted by adding of 5% formic acid followed by 100% acetonitrile. The supernatant was collected and transferred to the first fraction. The gel pieces were finally washed with acetonitrile for 10 min and the three pooled fractions were dried in speed-Vac.

### LC-MS/MS Analysis of N glycan site occupancy

Analysis of peptide readout was performed on a Q-exactive HF, Mass Spectrometer coupled with a Dionex Ultimate 3000 RS. Samples were reconstituted in 1% formic acid and aliquots of each sample were loaded at 10 µL/min onto a trap column equilibrated in 0.1% TFA. The peptides were eluted from the column at a constant flow rate of 300 nl/min and the column was kept at a constant temperature of 50°C.

Q-exactive HF was operated in data dependent positive ionization mode. Three blanks were run between each sample to reduce carry-over and mass accuracy checked before the start of sample analysis.

### Database search, protein, and peptide identifications

Raw MS data were searched against Human uniport proteome using the MASCOT search engine (Matrix Science, Version 2.6). Protein identifications and peptides were exported into Microsoft Excel.

### Example 3: Stable expression of OST enzymes in CHO-S cells

Bacterial artificial chromosomes (BACs) containing the *T. brucei* OST nucleic acid sequences were used to randomly integrate OST genes into the CHO-S genome resulting in stable expression of the *T. brucei* OST enzymes. Briefly, BACs are linearized and transfected into CHO-S cells using an Amaxa nucleofector. Antibiotic selection is performed for 7 days followed by separation of live and dead cells using fluorescent-assisted cell sorting (FACS). Single live cells are isolated after which single-cell clones are recovered and expanded. The clones are monitored for cell viability, gene copy numbers, titer and glycosylation patterns. The stable cell lines currently being produced can be found in Table 1 below.

**Table 1: cell lines for stable expression of OST enzymes.**

| **Cell line** | **Genes integrated** |
|---|---|
| CHO-S | *T. brucei* enzymes A, B & C |
| CHO-S | Erythropoietin (EPO) |
| CHO-S | ENPP1-Fc |
| CHO-S | sgp130-Fc (Olamkicept) |
| CHO-S | *T. brucei* enzymes A, B & C + EPO |
| CHO-S | *T. brucei* enzymes A, B & C + ENPP1-Fc |
| CHO-S | *T. brucei* enzymes A, B & C + sgp130-Fc (Olamkicept) |

### Transient expression of OST enzymes in Expi293 cells

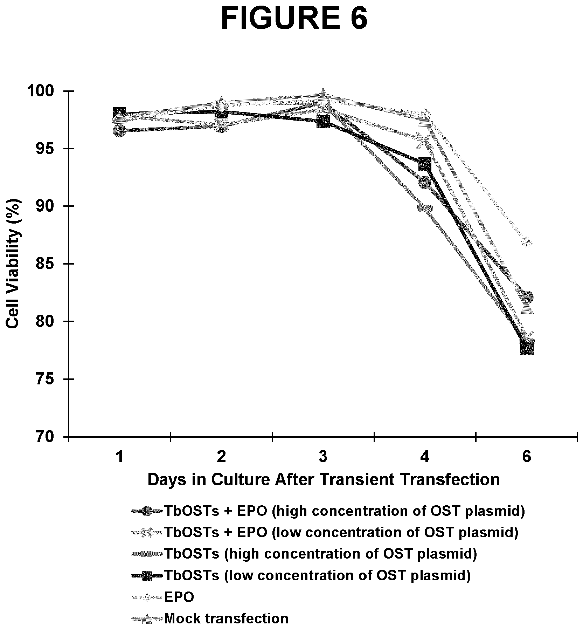

Mammalian expression plasmids containing the *T. brucei* OST nucleic acid sequences were used to transiently express OST enzymes in Expi293 cells, derived from the HEK293 cell line. Briefly, the plasmids were transfected into Expi293 cells using Expifectamine reagent from the Expi293 Expression System. Constitutive expression of the OST enzymes occurred for 6 days during which the cell viability of the Expi293 cells were monitored (Figure 6). The results indicate that expression of the OST cell lines in the mammalian derived Expi293 cell line does not negatively impact cell viability.

### SEQUENCES FORMING PART OF THE DESCRIPTION

### SEQ ID NO: 1 - consensus DNA sequence of active region of TbSTT3A

CGCGTCCGTGCGTTGTTCGTGAAACATACGCGTACCGGAAATCCCCTCGTGGATTCTGT
GGCTGAGCAT

### SEQ ID NO: 2 - DNA sequence of active region of TbSTT3A

AAACCTACAGCGTACCGCGTCCGTGCGTTGTTCGTGAAACATACGCGTACCGGAAATCC
CCTCGTGGATTCTGTGGCTGAGCATCGGCCGACGACTGCCGGGGCGTATCTGCGCTACT
TT

### SEQ ID NO: 3 - DNA sequence of active region of TbSTT3A (CHO optimised)

AAGCCTACCGCCTACAGAGTGCGGGCCCTGTTTGTGAAGCACACCAGAACCGGCAATCC
TCTGGTGGACTCTGTGGCCGAGCATAGACCTACAACCGCTGGCGCCTACCTGCGGTACT
TT

### SEQ ID NO: 4 - DNA sequence of active region of TbSTT3B

AGGCCGTTTTCTTCTCGTGTCCGTGCGTTGTTCGTGAAACATACGCGTACCGGAAATCC
CCTCGTGGATTCTGTGGCTGAGCACCATCCGGCGTCGAATGATGATTTCTTTGGTTACC
TT

### SEQ ID NO: 5 - DNA sequence of active region of TbSTT3B (CHO optimised)

CGGCCTTTCAGCTCTAGAGTGCGGGCCCTGTTTGTGAAGCACACCAGAACCGGCAATCC
TCTGGTGGACTCTGTGGCCGAGCACCATCCTGCCTCTAACGACGACTTCTTCGGCTACC
TG

### SEQ ID NO: 6 - DNA sequence of active region of TbSTT3C

AGGTCGTTTTCTTCTCGTGTCCGTGCGTTGTTCGTGAAACATACGCGTACCGGAAATCC
CCTCGTGGATTCTGTGGCTGAGCATCGGCCGACGACTGCCGGGGCCTTCCTACGTCATC
TT

### SEQ ID NO: 7 - DNA sequence of active region of TbSTT3C (CHO optimised)

CGGTCCTTCAGCTCTAGAGTGCGGGCCCTGTTTGTGAAGCACACCAGAACCGGCAATCC
TCTGGTGGACTCTGTGGCCGAGCATCGGCCTACAACAGCTGGCGCCTTTCTGAGACATC
TG

### SEQ ID NO: 8 - DNA sequence of full length TbSTT3A

ATGACGAAAGGTGGGAAAGTAGCTGTGACTAAGGGCTCAGCACAGAGTGATGGTGCTGG
TGAGGGAGGGATGAGTAAGGCCAAGTCATCCACTACGTTCGTCGCCACTGGCGGTGGTT
CTTTGCCTGCCTGGGCGCTAAAGGCTGTAAGCACGATTGTGAGTGCAGTGATTCTTATA
TACTCTGTCCATCGTGCTTACGATATACGACTTACTTCTGTCCGTCTTTATGGTGAGCT
TATTCACGAGTTCGACCCTTGGTTCAATTACCGTGCAACGCAGTACCTCAGCGACAACG
GGTGGCGTGCTTTTTTCCAATGGTACGACTACATGAGCTGGTACCCGCTTGGCCGACCG
GTGGGCACAACCATCTTCCCCGGAATGCAGCTTACCGGTGTAGCCATTCATCGTGTGCT
GGAAATGCTCGGGCGAGGTATGTCCATCAACAATATCTGTGTGTACATTCCTGCATGGT
TCGGTAGTATTGCCACTGTGTTGGCTGCTCTCATTGCGTACGAATCATCTAATTCGCTC
AGTGTCATGGCGTTTACTGCGTACTTTTTTTCCATCGTACCTGCACACCTGATGCGATC
AATGGCTGGTGAATTTGACAATGAGTGTGTTGCAATGGCGGCGATGCTTCTGACGTTCT
ACATGTGGGTACGATCGTTACGCAGCTCAAGTTCGTGGCCCATTGGCGCTTTAGCTGGT
GTGGCATACGGGTACATGGTGTCCACGTGGGGTGGTTATATTTTCGTGCTGAACATGGT
AGCCTTCCACGCTTCTGTATGTGTACTGCTTGATTGGGCTCGTGGGATATACAGCGTCA
GTTTGCTGAGGGCGTATTCACTGTTTTTTGTCATTGGCACTGCCCTTGCGATTTGCGTA
CCGCCAGTGGAGTGGACGCCCTTCCGGTCGCTGGAGCAACTGACAGCATTGTTTGTCTT
CGTTTTCATGTGGGCACTCCACTACTCGGAATACCTGCGTGAGCGTGCCCGAGCGCCCA
TTCACTCTTCTAAAGCACTTCAGATCCGTGCCCGCATTTTCATGGGCACCCTCTCCTTG
CTGTTGATTGTGGCAAGTTTGCTTGCCCCGTTCGGATTCTTCAAACCTACAGCGTACCG
CGTCCGTGCGTTGTTCGTGAAACATACGCGTACCGGAAATCCCCTCGTGGATTCTGTGG
CTGAGCATCGGCCGACGACTGCCGGGGCGTATCTGCGCTACTTTCATGTTTGTTACCCT
CTTTGGGGGTGCGGTGGGCTCTCTATGTTGGTATTCATGAAAAAGGACCGCTGGCGCGC
CATTGTTTTTCTTGCTTCACTTTCCACTGTTACGATGTATTTCAGCGCCCGTATGTCGC
GATTACTTCTGTTAGCGGGTCCCGCAGCAACGGCTTGCGCCGGCATGTTCATAGGGGGG
CTTTTTGATCTGGCGCTGTCACAGTTTGGTGATTTGCATAGCCCAAAAGATGCCTCCGG
CGATTCCGATCCCGCGGGAGGGTCGAAGCGGGCAAAGGGCAAAGTTGTTAATGAGCCTT
CCAAAAGAGCCATCTTTAGTCACCGCTGGTTTCAACGTTTAGTGCAATCGTTGCCCGTC
CCGCTACGACGTGGTATCGCGGTTGTGGTGCTCGTATGTCTCTTCGCCAATCCCATGAG
ACACTCATTCGAAAAGTCCTGCGAGAAAATGGCACATGCACTTTCATCTCCAAGGATCA
TTGCCGTGACTGATCTACCCAATGGAGAGAGAGTCCTCGCCGATGATTACTACGTGTCG
TACTTGTGGCTGCGAAACAATACGCCTGAAGATGCCCGTATTCTCTCATGGTGGGACTA

CGGGTATCAAATCACTGGAATTGGCAATCGCACAACCCTTGCGGACGGTAACACATGGA
GTCACAAGCACATAGCAACTATTGGGAAGATGCTTACATCCCCTGTGAAGGAGTCACAT
GCTCTTATACGCCATCTCGCTGATTATGTGCTGATATGGGCCGGTGAGGATCGGGGCGA
TTTACTTAAATCGCCACACATGGCTCGGATAGGCAACAGTGTATATCGCGATATGTGTT
CAGAAGACGATCCTAGATGCAGGCAGTTCGGCTTTGAGGGAGGTGACCTCAATAAGCCT
ACGCCTATGATGCAGCGGTCCCTATTATACAATCTGCACAGGTTTGGTACGGATGGCGG
GAAGACACAACTGGATAAGAACATGTTTCAGCTCGCCTACGTGTCAAAGTATGGTTTGG
TGAAGATCTACAAGGTGGTGAATGTGAGTGAAGAGAGCAAGGCGTGGGTCGCAGACCCA
AAGAACCGCGTATGCGACCCGCCCGGATCTTGGATATGCGCCGGCCAGTACCCGCCAGC
GAAGGAGATCCAAGACATGTTAGCGAAGCGGTTCCATTACGAATGA

### SEQ ID NO: 9 - DNA sequence of full length TbSTT3A (CHO optimised)

ATGACCAAAGGCGGAAAAGTGGCTGTGACCAAGGGCTCTGCTCAGTCTGATGGTGCTGG
CGAAGGCGGCATGTCTAAGGCCAAGTCCTCTACCACCTTCGTGGCTACCGGCGGAGGAT
CTTTGCCTGCTTGGGCTCTGAAGGCCGTGTCCACCATTGTGTCTGCCGTGATCCTGATC
TACTCCGTGCACCGGGCCTACGACATCAGGCTGACATCTGTCAGACTGTACGGCGAGCT
GATCCACGAGTTCGACCCCTGGTTCAACTACCGGGCTACCCAGTACCTGTCCGATAATG
GCTGGCGGGCCTTCTTCCAGTGGTACGACTATATGTCCTGGTATCCCCTGGGCAGACCC
GTGGGCACCACAATCTTTCCAGGCATGCAGCTGACAGGCGTGGCCATCCATAGAGTGCT
GGAAATGCTCGGCAGAGGCATGTCCATCAACAACATCTGCGTGTACATCCCCGCTTGGT
TTGGCTCTATCGCCACCGTTCTGGCCGCTCTGATCGCCTACGAGTCCTCCAACTCTCTG
TCCGTGATGGCCTTCACCGCCTACTTTTTCTCCATCGTGCCCGCTCACCTGATGCGGTC
TATGGCCGGCGAGTTCGATAACGAGTGTGTGGCCATGGCTGCCATGCTGCTGACCTTCT
ATATGTGGGTCCGAAGCCTGCGGTCCTCCTCCAGTTGGCCTATTGGAGCTTTGGCCGGC
GTGGCCTACGGCTACATGGTTTCTACCTGGGGCGGCTACATCTTCGTGCTGAACATGGT
GGCCTTCCACGCCTCTGTGTGCGTGCTGTTGGATTGGGCCAGAGGCATCTACAGCGTGT
CCCTGCTGAGAGCCTACAGCCTGTTCTTCGTGATCGGCACAGCCCTGGCCATCTGTGTG
CCACCTGTTGAGTGGACCCCTTTCAGATCCCTGGAACAGCTGACCGCTCTGTTCGTGTT
TGTGTTCATGTGGGCCCTGCACTACTCCGAGTACCTGAGAGAGAGAGCCAGGGCTCCCA
TCCACTCTTCTAAGGCCCTGCAGATCCGGGCCAGAATCTTCATGGGCACCCTGAGCCTG
CTGCTGATCGTGGCTTCTCTGCTGGCCCCTTTCGGCTTCTTTAAGCCTACCGCCTACAG

AGTGCGGGCCCTGTTTGTGAAGCACACCAGAACCGGCAATCCTCTGGTGGACTCTGTGG
CCGAGCATAGACCTACAACCGCTGGCGCCTACCTGCGGTACTTTCACGTGTGCTATCCT
CTGTGGGGCTGTGGCGGACTGTCCATGCTGGTGTTCATGAAGAAAGACCGGTGGCGGGC
CATCGTGTTTCTGGCTAGTCTGTCCACCGTGACCATGTACTTCAGCGCCCGGATGTCCA
GACTGCTGCTGTTGGCTGGACCTGCCGCTACAGCTTGTGCCGGCATGTTTATCGGCGGC
CTGTTCGATCTGGCCCTGTCTCAGTTTGGCGACCTGCACTCTCCTAAGGACGCCTCTGG
CGACTCTGATCCTGCTGGCGGATCCAAGCGCGCTAAGGGCAAAGTGGTCAACGAGCCTT
CCAAGAGAGCCATCTTCTCTCACCGGTGGTTCCAGCGGCTGGTGCAGTCTCTTCCAGTT
CCTCTGAGAAGAGGAATCGCCGTGGTGGTGCTCGTGTGCCTGTTCGCCAATCCTATGCG
GCACTCCTTCGAGAAGTCCTGCGAGAAAATGGCCCACGCTCTGAGCAGCCCTCGGATCA
TTGCTGTGACCGATCTGCCCAACGGCGAGCGAGTTCTGGCTGACGATTACTACGTGTCC
TACCTGTGGCTGCGGAACAACACCCCTGAGGACGCCAGAATCCTGTCTTGGTGGGATTA
CGGCTACCAGATCACCGGCATCGGCAACAGAACCACACTGGCCGATGGCAACACATGGT
CCCACAAGCACATTGCCACCATCGGCAAGATGCTGACCTCTCCTGTGAAAGAGTCTCAC
GCCCTGATCAGACACCTGGCCGACTACGTGTTGATCTGGGCTGGGGAAGATCGGGGCGA
TCTGCTGAAGTCTCCTCACATGGCCAGGATCGGCAACTCTGTGTACCGGGACATGTGCT
CCGAGGACGACCCTAGATGTCGGCAGTTCGGATTTGAAGGCGGCGACCTGAACAAGCCC
ACACCTATGATGCAGAGATCCCTGCTGTACAACCTGCACAGATTCGGCACCGATGGCGG
CAAGACCCAGCTGGACAAGAACATGTTTCAGCTGGCCTACGTCAGCAAATACGGCCTGG
TCAAGATCTACAAGGTCGTGAACGTGTCCGAGGAATCCAAGGCCTGGGTCGCCGATCCT
AAGAATAGAGTGTGCGACCCTCCTGGCTCCTGGATCTGTGCTGGACAGTACCCTCCTGC
CAAAGAAATCCAGGACATGCTGGCCAAGCGGTTCCACTACGAATGA

### SEQ ID NO: 10 - DNA sequence of full length TbSTT3B

ATGACGAAAGGTGGGAAAGTAGCTGTGACTAAGGGCTCAGCACAGAGTGATGGTGCTGG
TGAGGGAGGGATGAGTAAGGCCAAGTCATCCACTACGTTCGTCGCCACTGGCGGTGGTT
CTTTGCCTGCCTGGGCGCTAAAGGCTGTAAGCACGGTTGTGAGTGCAGTGATTCTTATA
TACTCTGTCCATCGTGCTTACGATATACGACTTACTTCTGTCCGTCTTTATGGTGAGCT
TATTCACGAGTTCGACCCTTGGTTCAATTACCGTGCAACGCAGTACCTCAGCGACAACG
GGTGGCGTGCTTTTTTCCAATGGTACGACTACATGAGCTGGTACCCGCTTGGCCGACCG
GTGGGCACAACCATCTTCCCCGGAATGCAGCTTACCGGTGTAGCCATTCATCGTGTGCT

GGAAATGCTCGGGCGAGGTATGTCCATCAACAATATCTGTGTGTACATTCCTGCATGGT
TCGGTAGTATTGCCACTGTGTTGGCTGCTCTCATTGCGTACGAATCATCTAATTCGCTC
AGTGTCATGGCGTTTACTGCGTACTTTTTTTCCATCGTACCTGCACACCTGATGCGATC
AATGGCTGGTGAATTTGACAATGAGTGTGTTGCAATGGCGGCGATGCTTCTGACGTTCT
ACATGTGGGTACGATCGTTACGCAGCTCAAGTTCGTGGCCCATTGGCGCTTTAGCTGGT
GTGGCATACGGGTACATGGTGTCCACGTGGGGTGGTTATATTTTCGTGCTGAACATGGT
AGCCTTCCACGCTTCTGTATGTGTACTGCTTGATTGGGCTCGTGGGACATACAGCGTCA
GTTTGCTGAGGGCGTATTCACTGTTTTTTGTCATTGGCACTGCCCTTGCGATTTGCGTA
CCACCAGTGGAGTGGACGCCCTTCCGGTCGCTGGAGCAACTGACAGCATTGTTTGTCTT
CGTTTTCATGTGGGCACTCCACTACTCGGAATACCTGCGTGAGCGTGCCCGAGCGCCCA
TTCACTCTTCTAAAGCACTTCAGATCCGTGCCCGCATTTTCATGGGCACCCTCTCCTTG
CTGTTGATTGTAGCTATCTACCTATTTTCGACAGGATACTTCAGGCCGTTTTCTTCTCG
TGTCCGTGCGTTGTTCGTGAAACATACGCGTACCGGAAATCCCCTCGTGGATTCTGTGG
CTGAGCACCATCCGGCGTCGAATGATGATTTCTTTGGTTACCTTCATGTATGTTACAAC
GGCTGGATAATTGGCTTTTTCTTCATGTCCGTGTCGTGCTTTTTCCATTGTACACCGGG
AATGTCGTTCCTGCTGTTGTACTCTATACTTGCGTACTACTTCTCTCTCAAGATGAGTC
GTCTGCTGTTACTTTCTGCACCTGTGGCTTCCATACTCACTGGCTATGTTGTGGGATCT
ATTGTTGACCTCGCAGCAGATTGTTTCGCCGCTTCCGGAACAGAGCACGCCGACAGTAA
GGAGCATCAAGGGAAAGCCCGTGGTAAGGGACAGAAGGAACAAATCACTGTCGAGTGTG
GGTGCCATAATCCCTTCTACAAATTATGGTGCAATTCATTTTCCTCCCGCCTGGTAGTT
GGTAAGTTTTTTGTCGTTGTTGTCCTTTCCATCTGTGGACCCACATTTCTTGGGTCTAA
CTTCCGGATATATTCTGAGCAATTCGCAGACAGCATGTCGAGCCCCCAGATTATAATGA
GGGCAACTGTCGGTGGACGACGAGTTATCTTGGATGATTACTACGTGTCGTACTTGTGG
CTGCGAAACAATACGCCTGAAGATGCCCGTATTCTCTCATGGTGGGACTACGGGTATCA
AATCACTGGAATTGGCAATCGCACAACCCTTGCGGATGGTAACACATGGAATCACGAGC
ACATAGCAACTATTGGGAAGATGCTTACATCCCCTGTGAAGGAGTCACATGCTCTTATA
CGCCATCTCGCTGATTATGTGCTGATATGGGCCGGTTATGATGGCAGCGATTTACTTAA
ATCGCCACACATGGCTCGGATAGGCAACAGTGTATATCGCGATATATGCTCAGAGGATG
ATCCGCTGTGTACGCAGTTCGGGTTTTATAGTGGTGACTTCAGTAAACCTACGCCTATG
ATGCAGCGGTCCCTATTATACAATCTGCACAGGTTTGGTACGGATGGCGGGAAGACACA
ACTGGATAAGAACATGTTTCAGCTCGCCTACGTGTCAAAGTATGGTTTGGTGAAGATCT

ACAAGGTGATGAATGTGAGTGAAGAGAGCAAGGCGTGGGTTGCAGACCCAAAGAACCGT
AAGTGCGATGCACCTGGATCTTGGATATGCACCGGCCAGTACCCGCCAGCGAAGGAGAT
CCAAGACATGTTAGCGAAGAGGATTGACTACGAACAACTCGAGGATTTCAACCGCCGCA
ATCGAAGTGACGCTTATTATCGTGCGTATATGCGTCAGATGGGTTAG

### SEQ ID NO: 11 - DNA sequence of full length TbSTT3B (CHO optimised)

ATGACCAAAGGCGGAAAAGTGGCTGTGACCAAGGGCTCTGCTCAGTCTGATGGTGCTGG
CGAAGGCGGCATGTCTAAGGCCAAGTCCTCTACCACCTTCGTGGCTACCGGCGGAGGAT
CTTTGCCTGCTTGGGCTCTGAAGGCCGTGTCCACAGTGGTGTCTGCCGTGATCCTGATC
TACTCTGTGCACCGGGCCTACGACATCCGGCTGACATCTGTCAGACTGTACGGCGAGCT
GATCCACGAGTTCGACCCCTGGTTCAACTACCGGGCTACCCAGTACCTGTCCGATAATG
GCTGGCGGGCCTTCTTCCAGTGGTACGACTATATGTCCTGGTATCCCCTGGGCAGACCC
GTGGGCACCACAATCTTTCCAGGCATGCAGCTGACAGGCGTGGCCATCCATAGAGTGCT
GGAAATGCTCGGCAGAGGCATGTCCATCAACAACATCTGCGTGTACATCCCCGCTTGGT
TTGGCTCTATCGCCACCGTTCTGGCCGCTCTGATCGCCTACGAGTCCTCCAACTCTCTG
TCCGTGATGGCCTTCACCGCCTACTTTTTCTCCATCGTGCCCGCTCACCTGATGCGGTC
TATGGCCGGCGAGTTCGATAACGAGTGTGTGGCCATGGCTGCCATGCTGCTGACCTTCT
ATATGTGGGTCCGAAGCCTGCGGTCCTCCTCCAGTTGGCCTATTGGAGCTTTGGCCGGC
GTGGCCTACGGCTACATGGTTTCTACCTGGGGCGGCTACATCTTCGTGCTGAACATGGT
GGCCTTCCACGCCTCTGTGTGCGTGCTGTTGGATTGGGCTAGAGGCACCTACTCCGTGT
CTCTGCTGAGAGCCTACAGCCTGTTCTTCGTGATCGGCACAGCCCTGGCCATCTGTGTG
CCACCTGTTGAGTGGACCCCTTTCAGATCCCTGGAACAGCTGACCGCTCTGTTCGTGTT
TGTGTTCATGTGGGCCCTGCACTACTCCGAGTACCTGAGAGAGAGAGCCAGGGCTCCCA
TCCACTCTTCTAAGGCCCTGCAGATCCGGGCCAGAATCTTCATGGGCACACTGTCCCTG
CTGCTGATCGTGGCTATCTACCTGTTCTCCACCGGCTACTTCCGGCCTTTCAGCTCTAG
AGTGCGGGCCCTGTTTGTGAAGCACACCAGAACCGGCAATCCTCTGGTGGACTCTGTGG
CCGAGCACCATCCTGCCTCTAACGACGACTTCTTCGGCTACCTGCACGTGTGCTACAAC
GGCTGGATCATCGGCTTTTTCTTTATGTCTGTGTCCTGCTTCTTCCACTGTACCCCTGG
CATGTCCTTTCTGCTGCTGTACTCCATCCTGGCCTACTACTTCAGCCTGAAGATGTCTA
GACTGCTGCTGCTCTCTGCCCCTGTGGCCTCTATCCTGACCGGATATGTCGTGGGCAGC
ATCGTGGATCTGGCCGCCGACTGTTTTGCTGCTTCTGGCACCGAGCACGCCGACTCCAA

AGAGCATCAGGGCAAAGCCAGAGGCAAGGGCCAGAAAGAGCAGATCACCGTCGAGTGCG
GCTGTCACAACCCCTTCTACAAGCTGTGGTGCAACTCCTTCTCCTCTCGGCTGGTCGTG
GGAAAGTTCTTTGTGGTGGTGGTGCTGTCTATCTGCGGCCCTACCTTCCTGGGCTCCAA
CTTCAGAATCTACAGCGAGCAGTTCGCCGACAGCATGTCTAGCCCTCAGATCATCATGA
GAGCCACCGTCGGCGGCAGAAGAGTGATCCTGGACGACTACTACGTGTCCTACCTGTGG
CTGCGGAACAACACCCCTGAGGACGCCAGAATCCTGTCTTGGTGGGATTACGGCTACCA
GATCACAGGCATCGGCAACAGAACCACACTGGCCGATGGCAACACCTGGAACCACGAGC
ACATTGCCACCATCGGCAAGATGCTGACCTCTCCTGTGAAAGAGTCTCACGCCCTGATC
AGACACCTGGCCGACTACGTGCTGATCTGGGCTGGCTACGATGGCTCCGACCTGCTGAA
GTCTCCTCACATGGCCAGGATCGGCAATTCCGTGTACCGGGACATCTGCTCCGAGGACG
ATCCTCTGTGTACCCAGTTCGGCTTCTACTCCGGCGACTTCTCCAAGCCTACACCTATG
ATGCAGCGGAGCCTGCTGTACAACCTGCACAGATTTGGCACCGATGGCGGCAAGACCCA
GCTGGACAAGAACATGTTTCAGCTGGCCTACGTCAGCAAATACGGCCTGGTCAAGATCT
ACAAAGTGATGAACGTGTCCGAGGAATCCAAGGCCTGGGTCGCCGATCCTAAGAACCGG
AAGTGTGACGCTCCTGGCAGCTGGATCTGCACCGGACAATACCCTCCAGCCAAAGAAAT
CCAGGACATGCTGGCCAAGCGGATCGACTACGAGCAGCTGGAAGATTTCAACCGGCGGA
ACAGATCCGACGCCTACTACCGCGCTTACATGCGGCAGATGGGATGA

### SEQ ID NO: 12 - DNA sequence of full length TbSTT3C

ATGACGAAAGGTGGGAAAGTAGCTGTGACTAAGGGCTCAGCACAGAGTGATGGTGCTGG
TGAGGGAGGGATGAGTAAGGCCAAGTCATCCACTACGTTCGTCGCCACTGGCGGTGGTT
CTTTGCCTGCCTGGGCGCTAAAGGCTGTAAGCACGGTTGTGAGTGCAGTGATTCTTATA
TACTCTGTCCATCGTGCTTACGATATACGACTTACTTCTGTCCGTCTTTATGGTGAGCT
TATTCACGAGTTCGACCCTTGGTTCAATTACCGTGCAACGCAGTACCTCAGCGACAACG
GGTGGCGTGCTTTTTTCCAATGGTACGACTACATGAGCTGGTACCCGCTTGGCCGACCG
GTGGGCACAACCATCTTCCCCGGAATGCAGCTTACCGGTGTAGCCATTCATCGTGTGCT
GGAAATGCTCGGGCGAGGTATGTCCATCAACAATATCTGTGTGTACATTCCTGCATGGT
TCGGTAGTATTGCCACTGTGTTGGCTGCTCTCATTGCGTACGAGTCATCTAATTCGCTC
AGTGTCATGGCGTTTACTGCGTACTTTTTTTCCATCGTACCTGCACACCTGATGCGATC
AATGGCTGGTGAATTTGACAATGAGTGTGTTGCAATGGCGGCGATGCTTCTGACGTTCT
ACATGTGGGTACGATCGTTACGCAGCTCAAGTTCGTGGCCCATTGGCGCTTTAGCTGGT

GTGGCATACGGGTACATGGTGTCCACGTGGGGTGGTTATATTTTCGTGCTGAACATGGT
AGCCTTCCACGCTTCTGTATGTGTACTGCTTGATTGGGCTCGTGGGACATACAGCGTCA
GTTTGCTGAGGGCGTATTCACTGTTTTTTGTCATTGGCACTGCCCTTGCGATTTGCGTA
CCACCAGTGGAGTGGACGCCCTTCCGGTCGCTGGAGCAACTGACAGCATTGTTTGTCTT
CGTTTTCATGTGGGCACTCCACTACTCGGAATACCTGCGTGAGCGTGCCCGAGCGCCCA
TTCACTCTTCTAAAGCACTTCAGATCCGTGCCCGCATTTTCATGGGCACCCTCTCCTTG
CTGTTGATTGTGGCTATCTACCTATTTTCGACAGGATACTTCAGGTCGTTTTCTTCTCG
TGTCCGTGCGTTGTTCGTGAAACATACGCGTACCGGAAATCCCCTCGTGGATTCTGTGG
CTGAGCATCGGCCGACGACTGCCGGGGCCTTCCTACGTCATCTTCATGTGTGTTACAAC
GGCTGGATAATTGGCTTTTTCTTCATGTCCGTGTCGTGCTTTTTCCATTGTACACCGGG
AATGTCGTTCCTGCTGTTGTACTCTATACTTGCGTACTACTTCTCTCTCAAGATGAGTC
GTCTGCTGTTACTTTCTGCACCTGTGGCTTCCATACTCACTGGCTATGTTGTGGGATCT
ATTGTTGACCTCGCAGCAGATTGTTTCGCCGCTTCCGGAACAGAGCACGCCGACAGTAA
GGAGCATCAAGGGAAAGCCCGTGGTAAGGGACAAAAGAGACAAATCACTGTCGAGTGTG
GGTGCCATAATCCCTTCTACAAATTATGGTGCAATTCATTTTCCTCCCGCCTGGTAGTT
GGTAAGTTTTTTGTCGTTGTTGTCCTTTCCATCTGTGGACCCACATTTCTTGGGTCTGA
GTTTAGGGCTCATTGCGAACGTTTCTCCGTAAGTGTTGCTAATCCGCGTATTATATCGA
GTATTAGGCACTCCGGTAAGTTGGTTCTTGCCGATGATTACTACGTGTCGTACTTGTGG
CTGCGAAACAATACGCCTGAAGATGCCCGTATTCTCTCATGGTGGGACTACGGGTATCA
AATCACTGGAATTGGCAATCGCACAACCCTTGCGGACGGTAACACATGGAATCACGAGC
ACATAGCAACTATTGGGAAGATGCTTACATCCCCTGTGAAGGAGTCACATGCTCTTATA
CGCCATCTCGCTGATTATGTGCTGATATGGGCCGGTGAGGATCGGGGCGATTTACGTAA
GTCACGGCATATGGCTCGGATAGGCAACAGTGTATATCGCGATATGTGTTCAGAAGACG
ATCCGCTGTGTACGCAGTTCGGGTTTTATAGTGGTGACTTCAATAAACCTACGCCTATG
ATGCAGCGGTCCCTATTATACAATCTGCACAGGTTTGGTACGGATGGCGGGAAGACACA
ACTGGATAAGAACATGTTTCAGCTCGCCTACGTGTCAAAGTATGGTTTGGTGAAGATCT
ACAAGGTGATGAATGTGAGTGAAGAGAGCAAGGCGTGGGTTGCAGACCCAAAGAACCGT
AAGTGCGATGCACCTGGATCTTGGATATGCGCCGGCCAGTACCCGCCAGCGAAGGAGAT
CCAAGACATGTTAGCGAAGAGGATTGACTACGAACAACTCGAGGATTTCAATCGCCGCA
ATCGAAGTGACGCTTATTATCGTGCGTATATGCGTCAGATGGGTTAG

### SEQ ID NO: 13 - DNA sequence of full length TbSTT3C (CHO optimised)

ATGACCAAAGGCGGAAAAGTGGCTGTGACCAAGGGCTCTGCTCAGTCTGATGGTGCTGG
CGAAGGCGGCATGTCTAAGGCCAAGTCCTCTACCACCTTCGTGGCTACCGGCGGAGGAT
CTTTGCCTGCTTGGGCTCTGAAGGCCGTGTCCACAGTGGTGTCTGCCGTGATCCTGATC
TACTCTGTGCACCGGGCCTACGACATCCGGCTGACATCTGTCAGACTGTACGGCGAGCT
GATCCACGAGTTCGACCCCTGGTTCAACTACCGGGCTACCCAGTACCTGTCCGATAATG
GCTGGCGGGCCTTCTTCCAGTGGTACGACTATATGTCCTGGTATCCCCTGGGCAGACCC
GTGGGCACCACAATCTTTCCAGGCATGCAGCTGACAGGCGTGGCCATCCATAGAGTGCT
GGAAATGCTCGGCAGAGGCATGTCCATCAACAACATCTGCGTGTACATCCCCGCTTGGT
TTGGCTCTATCGCCACCGTTCTGGCCGCTCTGATCGCCTACGAGTCCTCCAACTCTCTG
TCCGTGATGGCCTTCACCGCCTACTTTTTCTCCATCGTGCCCGCTCACCTGATGCGGTC
TATGGCCGGCGAGTTCGATAACGAGTGTGTGGCCATGGCTGCCATGCTGCTGACCTTCT
ATATGTGGGTCCGAAGCCTGCGGTCCTCCTCCAGTTGGCCTATTGGAGCTTTGGCCGGC
GTGGCCTACGGCTACATGGTTTCTACCTGGGGCGGCTACATCTTCGTGCTGAACATGGT
GGCCTTCCACGCCTCTGTGTGCGTGCTGTTGGATTGGGCTAGAGGCACCTACTCCGTGT
CTCTGCTGAGAGCCTACAGCCTGTTCTTCGTGATCGGCACAGCCCTGGCCATCTGTGTG
CCACCTGTTGAGTGGACCCCTTTCAGATCCCTGGAACAGCTGACCGCTCTGTTCGTGTT
TGTGTTCATGTGGGCCCTGCACTACTCCGAGTACCTGAGAGAGAGAGCCAGGGCTCCCA
TCCACTCTTCTAAGGCCCTGCAGATCCGGGCCAGAATCTTCATGGGCACACTGTCCCTG
CTGCTGATCGTGGCTATCTACCTGTTCTCCACCGGCTACTTCCGGTCCTTCAGCTCTAG
AGTGCGGGCCCTGTTTGTGAAGCACACCAGAACCGGCAATCCTCTGGTGGACTCTGTGG
CCGAGCATCGGCCTACAACAGCTGGCGCCTTTCTGAGACATCTGCACGTGTGCTACAAC
GGCTGGATCATCGGCTTTTTCTTTATGTCTGTGTCCTGCTTCTTCCACTGTACCCCTGG
CATGTCCTTTCTGCTGCTGTACTCCATCCTGGCCTACTACTTCAGCCTGAAGATGTCTA
GACTGCTGCTGCTCTCTGCCCCTGTGGCCTCTATCCTGACCGGATATGTCGTGGGCAGC
ATCGTGGATCTGGCCGCCGACTGTTTTGCTGCTTCTGGCACCGAGCACGCCGACTCCAA
AGAGCATCAGGGCAAAGCCAGAGGCAAGGGCCAGAAAAGACAGATCACCGTGGAATGCG
GCTGTCACAACCCCTTCTACAAGCTGTGGTGCAACTCCTTCTCCTCTCGGCTGGTCGTG
GGAAAGTTCTTTGTGGTGGTGGTGCTGTCTATCTGCGGCCCTACCTTTCTGGGCTCCGA
GTTTAGAGCCCACTGCGAGAGATTCTCCGTGTCCGTGGCCAATCCTCGGATCATCTCCT
CCATCCGGCACTCCGGAAAGCTGGTCCTGGCCGACGACTACTACGTGTCCTACCTGTGG
CTGCGGAACAACACCCCTGAGGACGCCAGAATCCTGTCTTGGTGGGATTACGGCTACCA

GATCACAGGCATCGGCAACAGAACCACACTGGCCGATGGCAACACCTGGAACCACGAGC
ACATTGCCACCATCGGCAAGATGCTGACCTCTCCTGTGAAAGAGTCTCACGCCCTGATC
CGGCACCTGGCCGATTATGTGTTGATCTGGGCTGGCGAGGACCGGGGCGATCTGAGAAA
GTCCAGACACATGGCTCGGATCGGCAACTCCGTGTACCGGGACATGTGCTCTGAGGACG
ACCCTCTGTGTACCCAGTTCGGCTTCTACTCCGGCGACTTCAACAAGCCCACACCTATG
ATGCAGCGGAGCCTGCTGTACAACCTGCACAGATTTGGCACCGACGGCGGCAAGACCCA
GCTGGACAAGAACATGTTTCAGCTGGCCTACGTCAGCAAATACGGCCTGGTCAAGATCT
ACAAAGTGATGAACGTGTCCGAGGAATCCAAGGCCTGGGTCGCCGATCCTAAGAACCGG
AAGTGTGACGCTCCTGGCTCCTGGATCTGTGCCGGACAATACCCTCCTGCCAAAGAAAT
CCAGGACATGCTGGCCAAGCGGATCGACTACGAGCAGCTGGAAGATTTCAACCGGCGGA
ACAGAAGCGACGCCTACTACCGCGCTTACATGCGGCAGATGGGATGA

### SEQ ID NO: 14 - amino acid sequence of active region of TbSTT3A

FFKPTAYRVRALFVKHTRTGNPLVDSVAEHRPTTAGAYLRYF

### SEQ ID NO: 15 - amino acid sequence of active region of TbSTT3B

YFRPFSSRVRALFVKHTRTGNPLVDSVAEHHPASNDDFFGYL

### SEQ ID NO: 16 - amino acid sequence of active region of TbSTT3C

YFRPFSSRVRALFVKHTRTGNPLVDSVAEHRPTTAGAFLRHL

### SEQ ID NO: 17 - amino acid sequence of full length TbSTT3A

MTKGGKVAVTKGSAQSDGAGEGGMSKAKSSTTFVATGGGSLPAWALKAVSTIVSAVILI
YSVHRAYDIRLTSVRLYGELIHEFDPWFNYRATQYLSDNGWRAFFQWYDYMSWYPLGRP
VGTTIFPGMQLTGVAIHRVLEMLGRGMSINNICVYIPAWFGSIATVLAALIAYESSNSL
SVMAFTAYFFSIVPAHLMRSMAGEFDNECVAMAAMLLTFYMWVRSLRSSSSWPIGALAG
VAYGYMVSTWGGYIFVLNMVAFHASVCVLLDWARGIYSVSLLRAYSLFFVIGTALAICV
PPVEWTPFRSLEQLTALFVFVFMWALHYSEYLRERARAPIHSSKALQIRARIFMGTLSL
LLIVASLLAPFGFFKPTAYRVRALFVKHTRTGNPLVDSVAEHRPTTAGAYLRYFHVCYP
LWGCGGLSMLVFMKKDRWRAIVFLASLSTVTMYFSARMSRLLLLAGPAATACAGMFIGG

LFDLALSQFGDLHSPKDASGDSDPAGGSKRAKGKVVNEPSKRAIFSHRWFQRLVQSLPV
PLRRGIAVVVLVCLFANPMRHSFEKSCEKMAHALSSPRIIAVTDLPNGERVLADDYYVS
YLWLRNNTPEDARILSWWDYGYQITGIGNRTTLADGNTWSHKHIATIGKMLTSPVKESH
ALIRHLADYVLIWAGEDRGDLLKSPHMARIGNSVYRDMCSEDDPRCRQFGFEGGDLNKP
TPMMQRSLLYNLHRFGTDGGKTQLDKNMFQLAYVSKYGLVKIYKVVNVSEESKAWVADP
KNRVCDPPGSWICAGQYPPAKEIQDMLAKRFHYE

### SEQ ID NO: 18 - amino acid sequence of full length TbSTT3B

MTKGGKVAVTKGSAQSDGAGEGGMSKAKSSTTFVATGGGSLPAWALKAVSTVVSAVILI
YSVHRAYDIRLTSVRLYGELIHEFDPWFNYRATQYLSDNGWRAFFQWYDYMSWYPLGRP
VGTTIFPGMQLTGVAIHRVLEMLGRGMSINNICVYIPAWFGSIATVLAALIAYESSNSL
SVMAFTAYFFSIVPAHLMRSMAGEFDNECVAMAAMLLTFYMWVRSLRSSSSWPIGALAG
VAYGYMVSTWGGYIFVLNMVAFHASVCVLLDWARGTYSVSLLRAYSLFFVIGTALAICV
PPVEWTPFRSLEQLTALFVFVFMWALHYSEYLRERARAPIHSSKALQIRARIFMGTLSL
LLIVAIYLFSTGYFRPFSSRVRALFVKHTRTGNPLVDSVAEHHPASNDDFFGYLHVCYN
GWIIGFFFMSVSCFFHCTPGMSFLLLYSILAYYFSLKMSRLLLLSAPVASILTGYVVGS
IVDLAADCFAASGTEHADSKEHQGKARGKGQKEQITVECGCHNPFYKLWCNSFSSRLVV
GKFFVVVVLSICGPTFLGSNFRIYSEQFADSMSSPQIIMRATVGGRRVILDDYYVSYLW
LRNNTPEDARILSWWDYGYQITGIGNRTTLADGNTWNHEHIATIGKMLTSPVKESHALI
RHLADYVLIWAGYDGSDLLKSPHMARIGNSVYRDICSEDDPLCTQFGFYSGDFSKPTPM
MQRSLLYNLHRFGTDGGKTQLDKNMFQLAYVSKYGLVKIYKVMNVSEESKAWVADPKNR
KCDAPGSWICTGQYPPAKEIQDMLAKRIDYEQLEDFNRRNRSDAYYRAYMRQMG

### SEQ ID NO: 19 - amino acid sequence of full length TbSTT3C

MTKGGKVAVTKGSAQSDGAGEGGMSKAKSSTTFVATGGGSLPAWALKAVSTVVSAVILI
YSVHRAYDIRLTSVRLYGELIHEFDPWFNYRATQYLSDNGWRAFFQWYDYMSWYPLGRP
VGTTIFPGMQLTGVAIHRVLEMLGRGMSINNICVYIPAWFGSIATVLAALIAYESSNSL
SVMAFTAYFFSIVPAHLMRSMAGEFDNECVAMAAMLLTFYMWVRSLRSSSSWPIGALAG
VAYGYMVSTWGGYIFVLNMVAFHASVCVLLDWARGTYSVSLLRAYSLFFVIGTALAICV
PPVEWTPFRSLEQLTALFVFVFMWALHYSEYLRERARAPIHSSKALQIRARIFMGTLSL
LLIVAIYLFSTGYFRSFSSRVRALFVKHTRTGNPLVDSVAEHRPTTAGAFLRHLHVCYN

GWIIGFFFMSVSCFFHCTPGMSFLLLYSILAYYFSLKMSRLLLLSAPVASILTGYVVGS
IVDLAADCFAASGTEHADSKEHQGKARGKGQKRQITVECGCHNPFYKLWCNSFSSRLVV
GKFFVVVVLSICGPTFLGSEFRAHCERFSVSVANPRIISSIRHSGKLVLADDYYVSYLW
LRNNTPEDARILSWWDYGYQITGIGNRTTLADGNTWNHEHIATIGKMLTSPVKESHALI
RHLADYVLIWAGEDRGDLRKSRHMARIGNSVYRDMCSEDDPLCTQFGFYSGDFNKPTPM
MQRSLLYNLHRFGTDGGKTQLDKNMFQLAYVSKYGLVKIYKVMNVSEESKAWVADPKNR
KCDAPGSWICAGQYPPAKEIQDMLAKRIDYEQLEDFNRRNRSDAYYRAYMRQMG

### SEQ ID NO: 20 - conserved amino acid residues in TbSTT3A, B and C active region

RVRALFVKHTRTGNPLVDSVAEH

### SEQ ID NO: 21 - consensus DNA sequence of active region of TbSTT3B

CGTGTCCGTGCGTTGTTCGTGAAACATACGCGTACCGGAAATCCCCTCGTGGATTCTGT
GGCTGAGCAC

### SEQ ID NO: 22 - consensus DNA sequence of active region of TbSTT3C

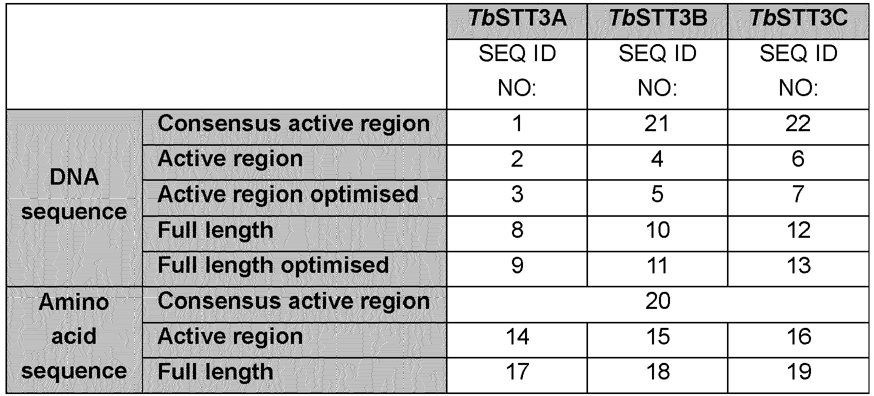

CGTGTCCGTGCGTTGTTCGTGAAACATACGCGTACCGGAAATCCCCTCGTGGATTCTGT
GGCTGAGCAT

### REFERENCES

Canada et al., Cell, 136(2): 272-283, 2010.
Cherepanova & Gilmore, Scientific Reports, 6(20946), 2016.
Delobel, Mass Spectrometry of Glycoproteins, pp 1-21, 2021.
Izquierdo, Mehlert, & Ferguson, Glycobiology, 22(5): 696-703, 2012.
Jinnelov et al., J Biol Chem, 292(49):20328-20341, 2017.
Kheller & Gilmore, Glycobiology, 16(4):47-62, 2005.
Kleizen & Braakman, Curr Opin Cell Biol, 16(4):343-9, 2004.
Mohanty et al., Biomolecules, 10(4):624, 2020.
Petrescu et al., Glycobiology, 14(2):103-14, 2004.
Pfeffer et al., Nature Communications, 5 (3072), 2014.
Reilly et al., Nature Reviews Nephrology, 15(346-366), 2019.

## Claims

1. A mammalian cell comprising at least one nucleic acid sequence encoding at least one oligosaccharyltransferase (OST) protein or functional fragment thereof, wherein the at least one OST protein is a *Trypanosoma* spp. OST protein, preferably wherein the at least one OST protein is a *Trypanosoma brucei* OST protein.

2. The mammalian cell according to claim 1, wherein the at least one nucleic acid sequence encoding the at least one OST protein or functional fragment thereof comprises a sequence according to any of SEQ ID NOs: 1, 2, 3, 4, 5, 6, 7, 8, 9, 10, 11, 12, 13, 21, and/or 22, or a sequence having at least 70% sequence identity thereto.

3. A mammalian cell comprising at least one nucleic acid sequence encoding at least one oligosaccharyltransferase (OST) protein or functional fragment thereof, wherein the at least one nucleic acid sequence encoding the at least one OST protein or functional fragment thereof comprises a sequence according to any of SEQ ID NOs: 1, 2, 3, 4, 5, 6, 7, 8, 9, 10, 11, 12, 13, 21, and/or 22, or a sequence having at least 70% sequence identity thereto.

4. A mammalian cell comprising at least one nucleic acid sequence encoding at least one oligosaccharyltransferase (OST) protein or functional fragment thereof, wherein the at least one OST protein comprises an amino acid sequence according to any of SEQ ID NOs: 14, 15, 16, 17, 18, 19 and/or 20 or a sequence having at least 70% sequence identity thereto.

5. The mammalian cell according to any one of claims 1 to 4, wherein the at least one nucleic acid sequence encoding the at least one OST protein or functional fragment thereof comprises the sequence according to SEQ ID NOs: 2, 3, 8 and/or 9, or a sequence having at least 70% identity to thereto, and wherein the cell further comprises a nucleic acid sequence encoding a further OST protein, or functional fragment thereof, and wherein the further nucleic acid sequence comprises a sequence according to SEQ ID NOs: 4, 5, 10 and/or 11, or a sequence having at least 70% identity to thereto, and further comprises the sequence according to SEQ ID NOs: 6, 7, 12 and/or 13, or a sequence having at least 70% identity to thereto.

6. The mammalian cell according to any preceding claim, wherein the at least one nucleic acid sequence encoding the at least one OST protein or functional fragment thereof comprises the sequence according to any of SEQ ID NOs: 4, 5, 10 and/or 11, or a sequence having at least 70% identity to thereto, and wherein the cell further comprises a nucleic acid sequence encoding a further OST protein, or functional fragment thereof, and wherein the further nucleic acid comprises a sequence according to any of SEQ ID NOs: 6, 7, 12 and/or 13, or a sequence having at least 70% identity to thereto.

7. The mammalian cell according to any one of claims 1 to 4, wherein the at least one nucleic acid sequence encoding the at least one OST protein or functional fragment thereof comprises the sequence according to any of SEQ ID NOs: 2, 3, 8 and/or 9, or a sequence having at least 70% identity to thereto, and wherein the cell further comprises a nucleic acid sequence encoding a further OST protein, or functional fragment thereof, and wherein the further nucleic acid sequence comprises a sequence according to any of SEQ ID NOs: 4, 5, 10 and/or 11, or a sequence having at least 70% identity to thereto.

8. The mammalian cell according to any one of claims 1 to 4, wherein the at least one nucleic acid sequence encoding the at least one OST protein or functional fragment thereof comprises the sequence according to any of SEQ ID NOs: 2, 3, 8 and/or 9, or a sequence having at least 70% identity to thereto, and wherein the cell further comprises a nucleic acid sequence encoding a further OST protein, or functional fragment thereof, and wherein the further nucleic acid sequence comprises a sequence according to any of SEQ ID NOs: 6, 7, 12 and/or 13, or a sequence having at least 70% identity to thereto.

9. An isolated nucleic acid molecule comprising a sequence according to any of SEQ ID NOs: 1, 2, 3, 4, 5, 6, 7, 8, 9, 10, 11, 12, 13, 21, and/or 22, or a sequence having at least 70% identity to thereto, preferably wherein the isolated nucleic acid molecule comprises a sequence according to SEQ ID NOs: 3, 5, 7, 9, 11 and/or 13.

10. A nucleic acid vector comprising at least one nucleic acid sequence according to any of SEQ ID NOs: 1, 2, 3, 4, 5, 6, 7, 8, 9, 10, 11, 12, 13, 21, and/or 22 or comprising at least one nucleic acid according to claim 9, preferably wherein the nucleic acid vector is selected from the group comprising, but not limited to, a plasmid-based expression vector, a bacterial artificial chromosome (BAC) vector, and a viral vector such as an adenoviral vector, adeno-associated vector (AAV), retroviral vector or lentiviral vector.

11. The mammalian cell according to any one of claims 1-8, wherein the mammalian cell is a Chinese hamster ovary (CHO) cell, a baby hamster kidney (BHK21) cell, a murine myeloma cell, or a human embryonic kidney (HEK293) cell, preferably wherein the mammalian cell is a Chinese hamster ovary (CHO) cell, more preferably wherein the CHO mammalian cell is a stable CHO (CHO-S) mammalian cell.

12. The mammalian cell according to claims 1-3, 5-8 or 11, wherein the OST protein or functional fragment thereof has an amino acid sequence according to SEQ ID NOs: 14, 15, 16, 17, 18, 19 and/or 20, or a sequence having at least 70% identity thereto, preferably wherein the OST protein is expressed by a mammalian cell as defined in any one of claims 1-3, 5-8 or 11.

13. The mammalian cell according to claim 11 or 12, wherein the mammalian cell is engineered to express a recombinant protein, preferably wherein the recombinant protein is a therapeutic protein.

14. A method of modifying the glycosylation profile of a recombinant protein, the method comprising contacting the recombinant protein with either (i) the mammalian cell according to any one of claims 1-8 or 11-13; or (ii) an OST protein or functional fragment thereof, wherein the OST protein is a *Trypanosoma* spp. OST protein, preferably wherein the OST protein is a *Trypanosoma brucei* OST protein, more preferably wherein the OST protein comprises an amino acid sequence according to any of SEQ ID NOs: 14, 15, 16, 17, 18, 19 and/or 20 ; or manufacturing the recombinant protein in a mammalian cell according to any one of claims 1-8 or 11-13, preferably wherein the mammalian cell is engineered to express the recombinant protein.

15. The method according to claim 14, wherein a frequency of N-glycan occupancy on the recombinant protein is altered, preferably wherein the frequency of N-glycan occupancy on the recombinant protein is increased; and/or wherein the recombinant protein is a protein selected from the group comprising a hormone, a cytokine, an antibody, an enzyme, a complement protein, a blood clotting factor, a functional fragment thereof, or any combination thereof.
